# EUROPEAN PATENT APPLICATION

(11) **EP 1 900 814 A1**
(43) Date of publication of application: **19.03.2008**
(21) Application number: 06757198.4
(22) Date of filing: 09.06.2006
(51) Int. Cl.: C12N 15/09, A61K 39/395, A61P 31/00, C07K 16/00

(54) **sc(Fv)2 SITE-DIRECTED MUTANT**

(30) Priority: 10.06.2005 JP 2005171673; 28.12.2005 JP 2005378639
(71) Applicant: CHUGAI SEIYAKU KABUSHIKI KAISHA, Tokyo, 115-8543 (JP)
(72) Inventor: IGAWA, Tomoyuki, CHUGAI SEIYAKU KABUSHIKI KAISHA, Gotenba-shi, Shizuoka 4128513 (JP); TSUNODA, Hiroyuki, CHUGAI SEIYAKU KABUSHIKI KAISHA, Gotenba-shi, Shizuoka4128513 (JP); KOBAYASHI, Takamitsu CHUGAI SEIYAKU K.K., Gotenba-shi, Shizuoka 4128513 (JP); KADONO, Shoujiro, CHUGAI SEIYAKU KABUSHIKI KAISHA, Gotenba-shi, Shizuoka 4128513 (JP)
(74) Representative: Vossius & Partner
(86) International application number: PCT/JP2006/311575
(87) International publication number: WO 2006/132341

(57) **Abstract**

To solve the above-mentioned problems, the present inventors introduced site-specific mutations into sc(Fv)2 and examined the stabilizing effects on sc(Fv)2. As a result, they succeeded for the first time in significantly increasing the Tm value of sc(Fv)2 by amino acid substitutions. Furthermore, they discovered that sc(Fv)2 is stabilized by introducing site-specific mutations into sc(Fv)2.

## Description

### Technical Field

The present invention relates to site-specific mutants of sc(Fv)2, a minibody (low-molecular-weight antibody), and uses thereof.

### Background Art

Developing and producing stable proteins with maintained functions and establishing their storage conditions are considered to be important in the formulation of biopharmaceuticals.

Proteins have different chemical properties from DNAs which handle genetic information, and their structures are flexible, which in other words means they are unstable. Even under physiological conditions, proteins are constantly in equilibrium between natural structure and disrupted structure (denatured structure).

Known pathways by which proteins generally degrade are: a degradation pathway accompanied by physical association of protein molecules such as formation of soluble multimers or production of precipitates/insoluble materials (Non-Patent Document 1); and a degradation pathway caused by chemical modifications through hydrolysis, deamidation reaction, methionine oxidation reaction, or such (Non-Patent Document 2). When developing proteins as pharmaceuticals, it is necessary to suppress both of these degradation pathways to a minimum and provide formulations in which the protein biological activity does not decrease during storage. Optimizing the pH of solutions, optimizing the type and concentration of buffers and salts, and optimizing the type and concentration of stabilizers are methods carried out for suppressing such degradation pathways to a minimum.

Known antibodies that can be used as pharmaceuticals are full-length antibodies, fragmented antibodies, minibodies, and such. It has been reported that a monomer-dimer equilibrium reaction takes place between two antibody molecules, and in antibody IgG molecules, monomers and dimers exist in a state of reversible equilibrium (Non-Patent Document 3). It is generally known that antibody molecules, including minibodies, readily aggregate and have very low stability (Non-Patent Document 4). When preparing antibody formulations, it is necessary to maintain antibodies in their monomeric state, which demonstrates activity in very high concentrations; therefore, formulating antibodies with secured stability has been considered a major challenge in developing antibodies as pharmaceuticals.

To develop protein pharmaceuticals having secured stability as pharmaceuticals, there are methods for increasing protein stability by optimizing formulation conditions such as those described above, and methods for enhancing the original stability of a protein by artificially introducing amino acid mutations to the primary sequence of a protein of interest. Various methods have been reported so far for improving the protein stability of a certain protein with known sequence by amino acid mutation (Non-Patent Documents 5, 6, and 7). For antibody molecules, sites (locations) of residues that strongly influence the stability in scFvs and stable amino acid residues for those locations have been reported from studies using scFvs which are single-chain antibodies of VH-VL (Non-Patent Documents 8, 9, 10, and 11). There are several reports that have actually improved the stability of scFv molecules by amino acid modification using these methods (Non-Patent Documents 12, 13, and 14).

Fab, Fv, scFv, sc(Fv)2, and such are known as antibody molecules with reduced molecular weights. Even for Fv and Fab which are fragmented antibody molecules from the same full-length IgG, Fab is known to have a different stability from Fv due to the presence of CH1 and CL. A similar situation holds for scFv: since CH1 and CL are absent in scFv, hydrophobic amino acids that are not exposed on the surface of Fab are exposed on the surface of scFv, causing its thermal stability to decrease; substitution of these residues by hydrophilic amino acids has been reported to improve stability in thermal acceleration assays (Non-Patent Document 7). Therefore, the sites (locations) of residues that influence stability and the stable residues are different in scFv and Fab, which are similar low-molecular-weight antibody molecules. Therefore, amino acid sites and amino acids affecting the stability of sc(Fv)2 may not necessarily match the amino acid sites and amino acids affecting the stability of scFvs that have been reported so far. To date, there are no reports on the sites (locations) of stability-affecting residues or stable residues in sc(Fv)2 molecules, and no investigation has been carried out so far for sc(Fv)2 to increase the stability of sc(Fv)2 by introducing site-specific amino acid mutations.
[Non-Patent Document 1] Int. J. Pharm., 2005, 289, 1-30.
[Non-Patent Document 2] Int. J. Pharm., 1999, 185, 129-188.
[Non-Patent Document 3] Biochemistry, 1999, 38, 13960-13967.
[Non-Patent Document 4] FEBS Letters, Volume 360, Issue 3 , 1995, 247-250.
[Non-Patent Document 5] Current Opinion in Biotechnology, 2002, 13, 333-337.
[Non-Patent Document 6] J. Biotechnology, 2004, 113, 105-120.
[Non-Patent Document 7] Microbiol Mol Biol Rev., 2001, 65(1), 1-43.
[Non-Patent Document 8] J. Mol. Biol., 2003, 325, 531-553.
[Non-Patent Document 9] J. Mol. Biol., 2001, 305, 989-1010.
[Non-Patent Document 10] Methods, 2004, 184-199.
[Non-Patent Document 11] Protein Eng., 1997, 10(4), 435-44.
[Non-Patent Document 12] Biochemistry, 2003, 42, 1517-1528.
[Non-Patent Document 13] Int. J. Cancer, 2003, 107, 822-829.
[Non-Patent Document 14] Protein Engineering, 1997, 10(12), 1453-1459.

### Disclosure of the Invention

### [Problems to be Solved by the Invention]

The present invention was achieved in view of the above circumstances. The present invention is aimed to provide methods for stabilizing sc(Fv)2 or methods for suppressing aggregation of sc(Fv)2 molecules, which comprise the step of introducing site-specific mutations into sc(Fv)2; to provide sc(Fv)2s that have been stabilized by the introduction of site-specific mutations; to stabilize sc(Fv)2 by allocating specific amino acids to sites that affect the stability of sc(Fv)2.; to provide sc(Fv)2 with an increased Tm value; and to provide pharmaceutical compositions comprising stabilized sc(Fv)2, methods for producing the pharmaceutical compositions, and kits comprising the pharmaceutical compositions.

### [Means for Solving the Problems]

To solve the above-mentioned problems, the present inventors introduced site-specific mutations into sc(Fv)2 and examined the stabilizing effects on sc(Fv)2.

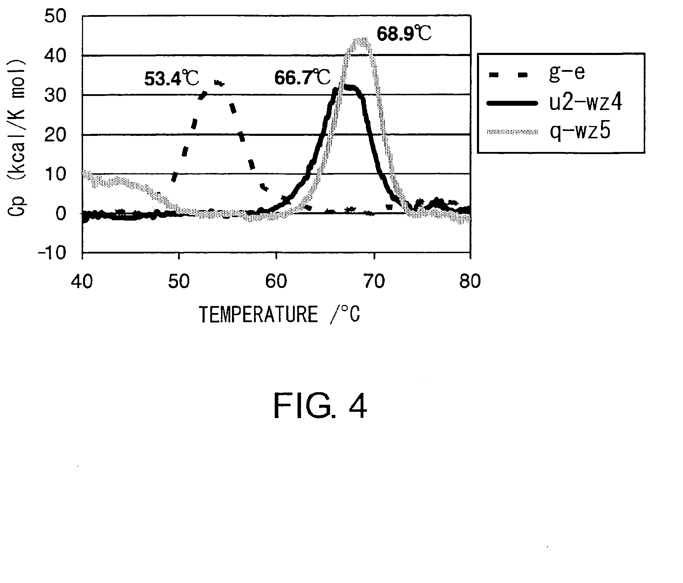

First, the present inventors measured the Tm values of humanized VB22B sc(Fv)2 site-specific mutants using Differential Scanning Calorimetry (DSC). As a result of carrying out amino acid modifications that increase the stability of hVB22B g-e sc(Fv)2, hVB22B u2-wz4 sc(Fv)2 whose Tm increased by 13.3°C and hVB22B q-wz5 whose Tm increased by 15.5°C were obtained (Fig. 4). To date, there are no reports on the Tm value of sc(Fv)2 or on increasing the Tm value by modifying the amino acids of sc(Fv)2.

Next, thermal acceleration assays were performed on sc(Fv)2 site-specific mutants, and the stability of each sc(Fv)2 site-specific mutant was evaluated based on the temporal change in the ratio of residual monomers after thermal acceleration, which is calculated by measuring the monomer area by gel filtration chromatographic (SEC) analysis.

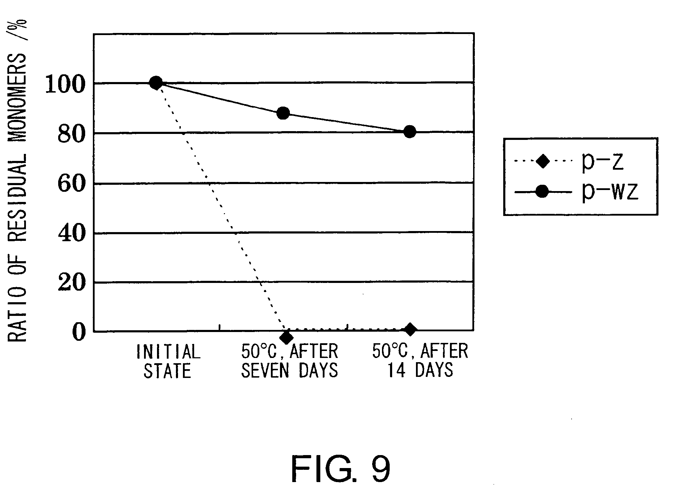

As a result, amino acid modifications that have stabilizing effects on sc(Fv)2 were discovered (Figs. 9-17 and 21-23).

Hence, through the present invention, the present inventors successfully increased the Tm of sc(Fv)2 by amino acid modification for the first time. Furthermore, the present inventors discovered that sc(Fv)2 is stabilized when site-specific mutations are introduced into sc(Fv)2, and thereby completed the present invention.

More specifically, the present invention provides the following:
[1] a method for stabilizing an sc(Fv)2, wherein the method comprises the step of introducing a site-specific mutation into the sc(Fv)2;
[2] a method for suppressing association between sc(Fv)2s, wherein the method comprises the step of introducing a site-specific mutation into the sc(Fv)2s;
[3] a method for increasing the Tm value of an sc(Fv)2 by 10°C or more, wherein the method comprises the step of introducing a site-specific mutation into the sc(Fv)2;
[4] the method of any one of [1] to [3], wherein the introduction of a site-specific mutation introduces a mutation to at least one amino acid selected from:
   (a) the 48th amino acid in the heavy chain;
   (b) the 65th amino acid in the heavy chain;
   (c) the 7th amino acid in the light chain;
   (d) the 8th amino acid in the light chain;
   (e) the 36th amino acid in the light chain;
   (f) the 43rd amino acid in the light chain;
   (g) the 45th amino acid in the light chain;
   (h) the 70th amino acid in the light chain;
   (i) the 81 st amino acid in the heavy chain;
   (j) the 39th amino acid in the heavy chain; and
   (k) the 38th amino acid in the light chain;
[5] the method of any one of [1] to [4], wherein the introduction of a site-specific mutation introduces at least one amino acid mutation selected from:
   (a) substitution of the 48th amino acid in the heavy chain to isoleucine;
   (b) substitution of the 65th amino acid in the heavy chain to glycine;
   (c) substitution of the 7th amino acid in the light chain to serine;
   (d) substitution of the 8th amino acid in the light chain to proline;
   (e) substitution of the 36th amino acid in the light chain to phenylalanine;
   (f) substitution of the 43rd amino acid in the light chain to alanine;
   (g) substitution of the 45th amino acid in the light chain to arginine;
   (h) substitution of the 70th amino acid in the light chain to aspartic acid;
   (i) substitution of the 81st amino acid in the heavy chain to glutamine;
   (j) substitution of the 39th amino acid in the heavy chain to glutamic acid or lysine; and
   (k) substitution of the 38th amino acid in the light chain to glutamic acid or lysine;
[6] a method for stabilizing an sc(Fv)2 by any one of the following methods:
   (a) a method for converting the 48th amino acid in the heavy chain to isoleucine;
   (b) a method for converting the 65th amino acid in the heavy chain to glycine;
   (c) a method for converting the 7th amino acid in the light chain to serine;
   (d) a method for converting the 8th amino acid in the light chain to proline;
   (e) a method for converting the 36th amino acid in the light chain to phenylalanine;
   (f) a method for converting the 43rd amino acid in the light chain to alanine;
   (g) a method for converting the 45th amino acid in the light chain to arginine;
   (h) a method for converting the 70th amino acid in the light chain to aspartic acid;
   (i) a method for converting the 81 st amino acid in the heavy chain to glutamine;
   (j) a method for converting the 39th amino acid in the heavy chain to glutamic acid or lysine; and
   (k) a method for converting the 38th amino acid in the light chain to glutamic acid or lysine;
[7] an sc(Fv)2 into which a mutation has been introduced to at least one amino acid selected from:
   (a) the 48th amino acid in the heavy chain;
   (b) the 65th amino acid in the heavy chain;
   (c) the 7th amino acid in the light chain;
   (d) the 8th amino acid in the light chain;
   (e) the 36th amino acid in the light chain;
   (f) the 43rd amino acid in the light chain;
   (g) the 45th amino acid in the light chain;
   (h) the 70th amino acid in the light chain;
   (i) the 81 st amino acid in the heavy chain;
   (j) the 39th amino acid in the heavy chain; and
   (k) the 38th amino acid in the light chain;
[8] an sc(Fv)2 into which at least one amino acid mutation selected from the following (a) to (k) has been introduced:
   (a) substitution of the 48th amino acid in the heavy chain to isoleucine;
   (b) substitution of the 65th amino acid in the heavy chain to glycine;
   (c) substitution of the 7th amino acid in the light chain to serine;
   (d) substitution of the 8th amino acid in the light chain to proline;
   (e) substitution of the 36th amino acid in the light chain to phenylalanine;
   (f) substitution of the 43rd amino acid in the light chain to alanine;
   (g) substitution of the 45th amino acid in the light chain to arginine;
   (h) substitution of the 70th amino acid in the light chain to aspartic acid;
   (i) substitution of the 81 st amino acid in the heavy chain to glutamine;
   (j) substitution of the 39th amino acid in the heavy chain to glutamic acid or lysine; and
   (k) substitution of the 38th amino acid in the light chain to glutamic acid or lysine;
[9] an sc(Fv)2 selected from:
   (a) an sc(Fv)2 with isoleucine as the 48th amino acid in the heavy chain;
   (b) an sc(Fv)2 with glycine as the 65th amino acid in the heavy chain;
   (c) an sc(Fv)2 with serine as the 7th amino acid in the light chain;
   (d) an sc(Fv)2 with proline as the 8th amino acid in the light chain;
   (e) an sc(Fv)2 with phenylalanine as the 36th amino acid in the light chain;
   (f) an sc(Fv)2 with alanine as the 43rd amino acid in the light chain;
   (g) an sc(Fv)2 with arginine as the 45th amino acid in the light chain;
   (h) an sc(Fv)2 with aspartic acid as the 70th amino acid in the light chain;
   (i) an sc(Fv)2 with glutamine as the 81 st amino acid in the heavy chain;
   (j) a method for converting the 39th amino acid in the heavy chain to glutamic acid or lysine; and
   (k) a method for converting the 38th amino acid in the light chain to glutamic acid or lysine;
[10] an sc(Fv)2 whose Tm value is 55°C or higher;
[11] an sc(Fv)2 whose Tm value has increased by 10°C or more by the introduction of a site-specific amino acid mutation, as compared with before the introduction;
[12] a pharmaceutical composition comprising the sc(Fv)2 of any one of [7] to [11]; and
[13] a method for producing the pharmaceutical composition of [12], wherein the method comprises the steps of:
   (1) introducing the site-specific mutation of any one of [1] to [5] into the sc(Fv)2; and
   (2) mixing with a pharmaceutically acceptable carrier.

### Brief Description of the Drawings

Fig. 1 shows the result of evaluating the agonistic activity of hVB22B g-e sc(Fv)2 using BaF-human Mpl.
Fig. 2 shows the result of evaluating the agonistic activity of hVB22B u2-wz4 sc(Fv)2 using BaF-human Mpl.
Fig. 3 shows the result of evaluating the agonistic activity of hVB22B q-wz5 sc(Fv)2 using BaF-human Mpl.
Fig. 4 shows the Tm values for hVB22B g-e sc(Fv)2 and the site-specific mutants of this sc(Fv)2.
Fig. 5 shows the change in the ratio of residual monomers, when Ile on site 37 in the heavy chain of sc(Fv)2 was substituted to Val.
Fig. 6 shows the change in the ratio of residual monomers when Pro on site 9 in the heavy chain of sc(Fv)2 was substituted to Ala.
Fig. 7 shows the change in the ratio of residual monomers when Pro on site 9 in the heavy chain of sc(Fv)2 was substituted to Ser.
Fig. 8 shows the change in the ratio of residual monomers when Leu on site 37 in the light chain of sc(Fv)2 was substituted to Gln.
Fig. 9 shows the change in the ratio of residual monomers when Ala on site 8 in the light chain of sc(Fv)2 was substituted to Pro.
Fig. 10 shows the change in the ratio of residual monomers when Val on site 65 in the heavy chain of sc(Fv)2 was substituted to Gly.
Fig. 11 shows the change in the ratio of residual monomers, when Ser on site 43 in the light chain of sc(Fv)2 was substituted to Ala and Gln on site 45 in the light chain of sc(Fv)2 was substituted to Arg.
Fig. 12 shows the change in the ratio of residual monomers when Tyr on site 36 in the light chain of sc(Fv)2 was substituted to Phe.
Fig. 13 shows the change in the ratio of residual monomers when Ala on site 70 in the light chain of sc(Fv)2 was substituted to Asp.
Fig. 14 shows the change in the ratio of residual monomers when Ala on site 7 in the light chain of sc(Fv)2 was substituted to Ser.
Fig. 15 shows the change in the ratio of residual monomers when Gln on site 81 in the heavy chain of sc(Fv)2 was substituted to Glu.
Fig. 16 shows the change in the ratio of residual monomers when Arg on site 81 in the heavy chain of sc(Fv)2 was substituted to Glu.
Fig. 17 shows the change in the ratio of residual monomers when Met on site 48 in sc(Fv)2 was substituted to Ile.
Fig. 18 shows the processes for generating the sc(Fv)2 gene.
Fig. 19-A shows the VH amino acid sequences of sc(Fv)2 used in the Examples. The - in the figure indicates that the amino acid sequence is the same as that of g-e.
Fig. 19-B shows continuation of the sequences in Fig. 19-A.
Fig. 20-A shows the VL amino acid sequences of sc(Fv)2 used in the Examples. The - in the figure indicates that the amino acid sequence is the same as that of g-e.
Fig. 20-B shows continuation of the sequences in Fig. 20-A.
Fig. 21 shows the results of gel filtration chromatography for u2-wz4, variant v1, and variant v3.
Fig. 22 shows the results of DSC analysis for u2-wz4-purified peak 1, u2-wz4-purified peak 2, variant v1, and variant v3.
Fig. 23 shows the results of gel filtration chromatographic analysis in thermal acceleration tests for u2-wz4-purified peak 1, u2-wz4-purified peak 2, variant v1, and variant v3.

### Best Mode for Carrying Out the Invention

The present inventors discovered that the stability of sc(Fv)2 increases by introducing site-specific mutations. The present inventors also discovered that the stability of sc(Fv)2 increases by arranging specific amino acids at specific sites. The present invention is based on these findings.

The present invention relates to methods for stabilizing sc(Fv)2, which comprises the step of introducing site-specific mutations into sc(Fv)2.

In the methods of the present invention, "modifying" and "introducing mutations" into amino acid residues specifically refer to substituting the original amino acid residues (before modification) with other amino acid residues, deleting the original amino acid residues, adding new amino acid resides, and such, but preferably refer to substituting the original amino acid residues with other amino acid residues. The original amino acid sequences (before modification) as used in the present invention may be naturally derived sequences, or sequences to which amino acid substitutions, humanization, or such have already been performed. In the present description, "modifying" amino acid residues and "introducing mutations" of amino acid residues have the same meaning.

In the present invention, "introducing mutations" of amino acid residues can be carried out by modifying sc(Fv)2-encoding DNAs.

In the present invention, when introducing mutations into the heavy chain (or light chain) of an sc(Fv)2, mutations may be introduced into both of the two heavy chains (or both of the two light chains) comprised in the sc(Fv)2, or mutations may be introduced into only one of the heavy chains (or light chains).

In the present invention, "modifying DNAs" means modifying DNAs according to amino acid residues introduced through "mutation introduction" in the present invention. More specifically, it refers to changing DNAs encoding the original amino acid residues into DNAs encoding amino acid residues introduced through modifications. Generally, it means performing gene manipulations or mutation treatments on original DNAs to insert, delete, or substitute at least one nucleotide to obtain codons encoding the amino acid residues of interest. In other words, codons encoding the original amino acid residues are substituted with codons encoding amino acid residues introduced through modifications. Such DNA modifications can be suitably carried out by those skilled in the art using known techniques such as site-specific mutagenesis or PCR mutagenesis.

In the present invention, the sites where the site-specific mutations are introduced are not particularly limited, and may be any site in sc(Fv)2, but are preferably any of the following sites:
(a) the 48th amino acid in the heavy chain;
(b) the 65th amino acid in the heavy chain;
(c) the 7th amino acid in the light chain;
(d) the 8th amino acid in the light chain;
(e) the 36th amino acid in the light chain;
(f) the 43rd amino acid in the light chain;
(g) the 45th amino acid in the light chain;
(h) the 70th amino acid in the light chain;
(i) the 81 st amino acid in the heavy chain;
(j) the 39th amino acid in the heavy chain; and
(k) the 38th amino acid in the light chain.

The amino acids after the substitution are not particularly limited, and any amino acid substitution is acceptable, but preferred examples of amino acids after substitution include the following amino acids:
(a) the 48th amino acid in the heavy chain: isoleucine;
(b) the 65th amino acid in the heavy chain: glycine;
(c) the 7th amino acid in the light chain: serine;
(d) the 8th amino acid in the light chain: proline;
(e) the 36th amino acid in the light chain: phenylalanine;
(f) the 43rd amino acid in the light chain: alanine;
(g) the 45th amino acid in the light chain: arginine;
(h) the 70th amino acid in the light chain: aspartic acid;
(i) the 81 st amino acid in the heavy chain: glutamine;
(j) the 39th amino acid in the heavy chain: glutamic acid or lysine; and
(k) the 38th amino acid in the light chain: glutamic acid or lysine.

Furthermore, the present invention relates to methods for stabilizing sc(Fv)2 by assigning specific amino acids to specific sites in sc(Fv)2. More specifically, it relates to methods for stabilizing sc(Fv)2 by any of the following methods:
(a) a method for converting the 48th amino acid in the heavy chain to isoleucine;
(b) a method for converting the 65th amino acid in the heavy chain to glycine;
(c) a method for converting the 7th amino acid in the light chain to serine;
(d) a method for converting the 8th amino acid in the light chain to proline;
(e) a method for converting the 36th amino acid in the light chain to phenylalanine;
(f) a method for converting the 43rd amino acid in the light chain to alanine;
(g) a method for converting the 45th amino acid in the light chain to arginine;
(h) a method for converting the 70th amino acid in the light chain to aspartic acid;
(i) a method for converting the 81 st amino acid in the heavy chain to glutamine;
(j) a method for converting the 39th amino acid in the heavy chain to glutamic acid or lysine; and
(k) a method for converting the 38th amino acid in the light chain to glutamic acid or lysine.

In the present invention, sc(Fv)2 is an antibody in which two heavy chain variable regions ([VH]) and two light chain variable regions ([VL]) are linked using linkers or such to produce a single chain polypeptide (Hudson et al., J. Immunol. Methods 1999; 231:177-189). sc(Fv)2 can be produced, for example, by linking two scFvs (single chain Fvs) (Huston, J. S. et al., Proc. Natl. Acad. Sci. U.S.A. (1988) 85, 5879-5883; and Pluckthun "The Pharmacology of Monoclonal Antibodies" Vol.113 Rosenburg and Moore ed. Springer Verlag, New York, pp. 269-315, 1994) with a linker or such. Arbitrary peptide linkers that can be introduced by genetic engineering, or synthetic linkers, for example, those disclosed in Protein Engineering, 9(3), 299-305, 1996 can be used as linkers, but in the present invention, peptide linkers are preferable. The length of the peptide linkers is not particularly limited, and can be suitably selected according to the purpose by those skilled in the art; however, the length is generally 1 to 100 amino acids, preferably 5 to 30 amino acids, and particularly preferably 12 to 18 amino acids (for example, 15 amino acids).

The order of the two heavy chain variable regions and the two light chain variable regions that are linked is not particularly limited, and they may be placed in any order. Examples include the following arrangements:
[VH]-linker-[VL]-linker-[VH]-linker [VL]
[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

In the present invention, sc(Fv)2 preferably has the [VH]-linker-[VL]-linker-[VH]-linker-[VL] arrangement.

Examples of amino acid sequences of the peptide linkers include the following sequences:
Ser
Gly-Ser
Gly-Gly-Ser
Ser-Gly-Gly
Gly-Gly-Gly-Ser (SEQ ID NO: 42)
Ser-Gly-Gly-Gly (SEQ ID NO: 43)
Gly-Gly-Gly-Gly-Ser (SEQ ID NO: 44)
Ser-Gly-Gly-Gly-Gly (SEQ ID NO: 45)
Gly-Gly-Gly-Gly-Gly-Ser (SEQ ID NO: 46)
Ser-Gly-Gly-Gly-Gly-Gly (SEQ ID NO: 47)
Gly-Gly-Gly-Gly-Gly-Gly-Ser (SEQ ID NO: 48)
Ser-Gly-Gly-Gly-Gly-Gly-Gly (SEQ ID NO: 49)
(Gly-Gly-Gly-Gly-Ser (SEQ ID NO: 44))n
(Ser-Gly-Gly-Gly-Gly (SEQ ID NO: 45))n
where n is an integer of 1 or larger.

Synthetic linkers (chemical crosslinking agents) include crosslinking agents routinely used to crosslink peptides, for example, N-hydroxy succinimide (NHS), disuccinimidyl suberate (DSS), bis(sulfosuccinimidyl) suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl] sulfone (BSOCOES), and bis[2-(sulfosuccinimidoxycarbonyloxy)ethyl] sulfone (sulfo-BSOCOES). These crosslinking agents are commercially available.

In general, three linkers are required to link four antibody variable regions together. The linkers to be used may be of the same type or different types.

Amino acid sequences of the heavy chain variable regions or light chain variable regions may comprise substitutions, deletions, additions, and/or insertions. Moreover, so long as the heavy chain variable and light chain variable regions have, when assembled, the antigen-binding activity, a part may be deleted or other peptides may be added. Furthermore, the variable regions may also be humanized.

Methods for preparing polypeptides functionally equivalent to a certain polypeptide are well known to those skilled in the art, and include methods of introducing mutations into polypeptides. For example, those skilled in the art can prepare an antibody functionally equivalent to the antibodies of the present invention by introducing appropriate mutations into the antibody using site-directed mutagenesis (Hashimoto-Gotoh, T. et al. Gene 152, 271-275, (1995); Zoller, MJ, and Smith, M. Methods Enzymol. 100, 468-500, (1983); Kramer, W. et al., Nucleic Acids Res. 12, 9441-9456, (1984); Kramer, W. and Fritz HJ, Methods Enzymol. 154, 350-367, (1987); Kunkel, TA, Proc. Natl. Acad. Sci. USA. 82, 488-492, (1985); Kunkel, Methods Enzymol. 85, 2763-2766, (1988)), or such. Amino acid mutations may occur naturally. Thus, the present invention also comprises antibodies functionally equivalent to the antibodies of the present invention and comprising the amino acid sequences of these antibodies, in which one or more amino acids are mutated.

The number of amino acids that are mutated is not particularly limited. Generally, the number is 30 amino acids or less, preferably 15 amino acids or less, more preferably five amino acids or less (for example, three amino acids or less). The amino acid residues to be mutated are preferably mutated to other amino acids in which the properties of the amino acid side chains are maintained. Examples of amino acid side chain properties are: hydrophobic amino acids (A, I, L, M, F, P, W, Y, and V), hydrophilic amino acids (R, D, N, C, E, Q, G, H, K, S, and T), amino acids comprising the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); aromatic ring-containing side chains (H, F, Y, and W) (amino acids are represented by one-letter codes in parentheses). A polypeptide comprising a modified amino acid sequence, in which one or more amino acid residues is deleted, added, and/or replaced with other amino acids, is known to retain its original biological activity (Mark, D. F. et al., Proc. Natl. Acad. Sci. USA 81, 5662-5666 (1984); Zoller, M. J. & Smith, M. Nucleic Acids Research 10, 6487-6500 (1982); Wang, A. et al., Science 224, 1431-1433; Dalbadie-McFarland, G et al., Proc. Natl. Acad. Sci. USA 79, 6409-6413 (1982)). Furthermore, the amino acid sequences of antibody constant regions are known to those skilled in the art.

Chimeric antibodies are antibodies generated by combining sequences derived from different animals, for example, antibodies comprising variable regions of mouse antibody heavy and light chains and constant regions of human antibody heavy and light chains. Chimeric antibodies can be produced by known methods, for example, by linking DNAs encoding an antibody V region and DNAs encoding a human antibody C region, incorporating this into an expression vector, introducing the vector into a host, and then producing the antibody.

Humanized antibodies are also referred to as reshaped human antibodies. They are antibodies in which the complementarity determining regions (CDRs) of an antibody of a non-human mammal, for example a mouse, have been transferred to the CDRs of a human antibody, and general genetic recombination procedures for this are also known (see European Patent Application No. 125023 and WO 96/02576).

Specifically, DNA sequences designed to link mouse antibody CDRs to the framework region (FR) of a human antibody are synthesized by PCR, using as primers a number of oligonucleotides produced to comprise overlapping portions for the terminal regions of both the CDRs and FR (see methods described in WO 98/13388).

The human antibody framework regions that form favorable antigen-binding sites with the complementarity determining regions are selected as the framework regions to be linked via the CDRs. Amino acids in the framework region of the antibody variable region may be substituted as required such that the CDRs of the reshaped human antibody form suitable antigen-binding sites (Sato, K. et al., Cancer Res. (1993) 53, 851-856).

Human antibody constant regions are generally used for the constant regions of chimeric and humanized antibodies, and for example, Cγ1, Cγ2, Cγ3, and Cγ4 can be used for the H chain and Cκ and Cλ can be used for the L chain.

Generally, chimeric antibodies comprise variable regions of an antibody derived from a non-human mammal and constant regions derived from a human antibody. On the other hand, humanized antibodies comprise complementarity determining regions of an antibody derived from a non-human animal, and framework regions and constant regions derived from a human antibody.

Amino acids in the variable regions (for example, FR) and constant regions can be, for example, substituted by other amino acids after producing the chimeric or humanized antibodies.

The origin of the variable regions in the chimeric antibodies or CDRs in the humanized antibodies is not particularly limited, and they may be derived from any animal. For example, sequences of mouse antibodies, rat antibodies, rabbit antibodies, camel antibodies, or such can be used.

sc(Fv)2 used in the present invention may be conjugated antibodies bound to various kinds of molecules such as polyethylene glycol (PEG), radioactive substances, and toxins. Such conjugated antibodies can be obtained by chemically modifying the obtained antibodies. Methods for modifying antibodies are already established in this field. These conjugated antibodies are also included in the sc(Fv)2 of the present invention.

sc(Fv)2 used in the present invention-may be bispecific antibodies (see for example, Journal of Immunology, 1994, 152, 5368-5374). Bispecific antibodies may recognize two different types of antigens or may recognize different epitopes on a same antigen.

sc(Fv)2 of the present invention may have a different protein, such as the Fc portion of IgG, fused to its N terminus or C terminus (Clinical Cancer Research, 2004, 10, 1274-1281). Proteins that are fused can be suitably selected by those skilled in the art.

sc(Fv)2 described above can be produced by methods known to those skilled in the art. Specifically, the DNA of an sc(Fv)2 of interest is incorporated into an expression vector. The DNA is incorporated into an expression vector such that it is expressed under the control of expression regulatory regions such as enhancers and promoters. Next, the antibody can be expressed by transforming host cells with the expression vector. Herein, suitable combinations of hosts and expression vectors can be used.

The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs.

In particular, when vectors are used to produce antibodies, expression vectors are useful. When an expression vector is expressed, for example, in *E. coli,* it should have the above characteristics in order to be amplified in *E. coli.* Additionally, when *E. coli,* such as JM109, DH5α, HB101, or XL1-Blue are used as the host, the vector must have a promoter that allows efficient expression of the desired gene in *E. coli,* for example, lacZ promoter (Ward et al. (1989) Nature 341:544-546; (1992) FASEB J. 6:2422-2427), araB promoter (Better et al. (1988) Science 240:1041-1043), or T7 promoter. Other examples of the vectors include pGEX-5X-1 (Pharmacia), "QIAexpress system" (QIAGEN), pEGFP, and pET (for which BL21, a strain expressing T7 RNA polymerase, is preferably used as the host).

Furthermore, the vectors may comprise a signal sequence for polypeptide secretion. When producing polypeptides into the periplasm of *E. coli,* the pelB signal sequence (Lei, S. P. et al. J. Bacteriol. 169:4379 (1987)) may be used as a signal sequence for polypeptide secretion. For example, calcium chloride methods or electroporation methods can be used to introduce vectors into host cells.

In addition to *E. coli,* examples of vectors for producing the polypeptides of the present invention include expression vectors derived from: mammals (e.g., pCDNA3 (Invitrogen), pEGF-BOS (Nucleic Acids Res. (1990) 18(17):5322), pEF, pCDM8); insect cells (e.g., "Bac-to-BAC baculovirus expression system" (GIBCO-BRL), pBacPAK8); plants (e.g., pMH1, pMH2); animal viruses (e.g., pHSV, pMV, pAdexLcw); retroviruses (e.g., pZIPneo); yeasts (e.g., "Pichia Expression Kit" (Invitrogen), pNV11, SP-Q01); and *Bacillus subtilis* (e.g., pPL608, pKTH50).

In order to express proteins in animal cells such as CHO, COS, and NIH3T3 cells, the vector must have a promoter necessary for expression in such cells, for example, an SV40 promoter (Mulligan et al. (1979) Nature 277:108), MMTV-LTR promoter, EF1α promoter (Mizushima et al. (1990) Nucleic Acids Res. 18:5322), CAG promoter (Gene (1991) 108:193), CMV promoter, etc.). It is further preferable that the vector also comprises a gene for selecting transformants (for example, a drug-resistance gene that allows selection by a drug such as neomycin and G418). Examples of vectors with such characteristics include pMAM, pDR2, pBK-RSV, pBK-CMV, pOPRSV, and pOP13.

In addition, to stably express a gene and amplify the gene copy number in cells, CHO cells that are defective in the nucleic acid synthesis pathway are introduced with a vector containing a DHFR gene (for example, pCHOI) to compensate for the defect, and the copy number is amplified using methotrexate (MTX). Alternatively, a COS cell, which carries an SV40 T antigen-expressing gene on its chromosome, can be transformed with a vector containing the SV40 replication origin (for example, pcD) for transient gene expression. The replication origins derived from polyoma virus, adenovirus, bovine papilloma virus (BPV), and such can be used. Furthermore, to increase the gene copy number in host cells, the expression vectors may comprise, as a selection marker, the aminoglycoside transferase (APH) gene, thymidine kinase (TK) gene, *E. coli* xanthine guanine phosphoribosyl transferase (Ecogpt) gene, dihydrofolate reductase (dhfr) gene, and such.

In the present invention, the term "stabilize" refers to suppressing aggregation caused by mutations. Suppression of aggregation does not have to be complete suppression of aggregation, and may simply be a decrease in the degree or percentage of aggregation. In the present invention, aggregation may be reversible or irreversible aggregation.

In the present invention, "aggregation" may be aggregation of sc(Fv)2 that takes place as time progresses, or it may be aggregation that takes place as sc(Fv)2 is produced in host cells or aggregation that takes place as sc(Fv)2 is secreted from host cells. Suppression of the decrease in sc(Fv)2 activity and suppression of the conversion to non-natural state are also considered to have the same meaning as the term "stabilization" of the present invention.

Whether stabilization has taken place or not can be measured by methods known to those skilled in the art. For example, whether aggregation was suppressed or not can be measured by methods described in the Examples. The degree of aggregation (percentage of aggregation) of antibody molecules can also be measured by methods known to those skilled in the art, such as the sedimentation equilibrium method (ultracentrifugation), osmotic pressure method, light scattering method, low-angle laser light scattering method, X-ray small angle scattering method, neutron small angle scattering method, or gel filtration method.

Examples of a method for measuring the degree of aggregation (percentage of aggregation) of antibody molecules include methods using size exclusion chromatography (SEC), but it is not limited thereto.

The Tm value is known to serve as an indicator of protein stability in solution. Generally, the higher the temperature, the more unstable the proteins; therefore, as proteins are heated, degeneration and aggregation start to take place at a certain temperature, and proteins completely degenerate or aggregate at another temperature. The Tm value is the midpoint temperature in such a change, and it can generally be measured by optical analyses such as differential scanning calorimetry (DSC), change in temperature-dependent CD spectra, or such. When developing proteins as pharmaceuticals, it is known that highly stable formulations can be produced by selecting formulation conditions that give high Tm values (Pharm. Res. 1998 Feb; 15(2):200-8). Therefore, it is thought to facilitate the development into pharmaceutical formulations by creating mutants whose Tm value is increased by amino acid modification.

For such reasons, in the present invention, when the Tm value of an sc(Fv)2 molecule is increased, the sc(Fv)2 can be considered to have been stabilized. Therefore, the present invention relates to methods for increasing the Tm value of an sc(Fv)2 by 10°C or more, where the methods comprise the step of introducing site-specific mutations into the sc(Fv)2.

Whether the Tm value has increased or not can be examined by comparing the Tm value before amino acid modification with the Tm value after amino acid modification. The increase in the Tm value is not particularly limited so long as the Tm value after amino acid modification is higher than the Tm value before amino acid modification, but the increase is preferably 10°C or more, more preferably 13°C or more, and particularly preferably 15°C or more. The upper limit of the Tm value is not particularly limited, but it is generally 150°C or so.

Tm values can be measured by methods known to those skilled in the art, and for example, they can be measured by methods described in the Examples.

The number of amino acids that are modified to increase the Tm value is not particularly limited, and a single amino acid may be modified or multiple amino acids may be modified.

In the present invention, stabilization of sc(Fv)2 may be a temporary stabilization of sc(Fv)2 molecules, or it may be an eventual stabilization of sc(Fv)2 molecules after a certain period of time. More specifically, it may be a temporary maintenance of the activities as an sc(Fv)2 composition, or it may be an eventual maintenance of the sc(Fv)2 molecule activities after a certain period of time.

In the present invention, the activities are not particularly limited and may be any activities such as binding activity, neutralizing activity, cytotoxic activity, agonistic activity, antagonistic activity, enzyme activity, but binding activity or agonistic activity is preferred.

Agonistic activity is an activity that induces some kind of change in physiological activity after the binding of an antibody to an antigen, such as a receptor, which leads to signal transduction and such in cells. Without limitation, examples of the physiological activity include proliferation activity, survival activity, differentiation activity, transcriptional activity, membrane transport activity, binding activity, proteolytic activity, phosphorylation/dephosphorylation activity, redox activity, transfer activity, nucleolytic activity, dehydration activity, cell death-inducing activity, and apoptosis-inducing activity.

In the present invention, the antigens are not particularly limited, and may be any type of antigen. Examples of antigens include receptors, cancer antigens, MHC antigens, and differentiation antigens. Examples of receptors include receptors belonging to receptor families such as hematopoietic factor receptor family, cytokine receptor family, tyrosine kinase-type receptor family, serine/threonine kinase-type receptor family, TNF receptor family, G-protein coupled receptor family, GPI anchored-type receptor family, tyrosine phosphatase-type receptor family, adhesion factor family, and hormone receptor family.

Examples of specific receptors belonging to the above-mentioned receptor families include human and mouse erythropoietin (EPO) receptors, human and mouse granulocyte-colony stimulating factor (G-CSF) receptors, human and mouse thrombopoietin (TPO) receptors, human and mouse insulin receptors, human and mouse Flt-3 ligand receptors, human and mouse platelet-derived growth factor (PDGF) receptors, human and mouse interferon (IFN)-α or β receptors, human and mouse leptin receptors, human and mouse growth hormone (GH) receptors, human and mouse interleukin (IL)-10 receptors, human and mouse insulin-like growth factor (IGF)-I receptors, human and mouse leukemia inhibitory factor (LIF) receptors, and human and mouse ciliary neurotrophic factor (CNTF) receptors.

Cancer antigens are antigens that are expressed as cells become malignant, and are also called tumor-specific antigens. Abnormal sugar chains that appear on cell surfaces or on protein molecules when cells become cancerous are also cancer antigens, and are specifically called sugar-chain cancer antigens. Examples of cancer antigens include CA19-9, CA15-3, and sialyl SSEA-1 (SLX).

MHC antigens are roughly classified into MHC class I antigens and MHC class II antigens. MHC class I antigens include HLA-A, -B, -C, -E, -F, -G, and -H, and MHC class II antigens include HLA-DR, -DQ, -and -DP.

Differentiation antigens include CD1, CD2, CD3, CD4, CD5, CD6, CD7, CD8, CD10, CD11a, CD11b, CD11c, CD13, CD14, CD15s, CD16, CD18, CD19, CD20, CD21, CD23, CD25, CD28, CD29, CD30, CD32, CD33, CD34, CD35, CD38, CD40, CD41a, CD41b, CD42a, CD42b, CD43, CD44, CD45, CD45RO, CD48, CD49a, CD49b, CD49c, CD49d, CD49e, CD49f, CD51, CD54, CD55, CD56, CD57, CD58, CD61, CD62E, CD62L, CD62P, CD64, CD69, CD71, CD73, CD95, CD102, CD106, CD122, CD126, CDw130.

Detection indicators used for measuring changes in activity can be used so long as quantitative and/or qualitative changes can be measured. For example, indicators for cell free systems (cell free assays), indicators for cell-based systems (cell-based assays), indicators for tissue-based systems, and indicators for biological systems can be used.

Enzymatic reactions, as well as quantitative and/or qualitative changes in proteins, DNAs, or RNAs can be used as indicators for cell free systems. For example, amino acid transfer reaction, sugar transfer reaction, dehydration reaction, dehydrogenation reaction, substrate cleaving-reaction, and such can be used for the enzymatic reactions. Protein phosphorylation, dephosphorylation, dimerization, multimerization, degradation, dissociation, and such, and DNA or RNA amplification, cleavage, and elongation can also be used. For example, phosphorylation of a protein present in the downstream of a signal transduction pathway can be used as a detection indicator.

Cell phenotypic changes, for example, quantitative and/or qualitative changes of produced substances, changes in proliferation activity, changes in cell number, changes in morphology, and changes in properties can be used as indicators for cell-based systems. Secretory proteins, surface antigens, intracellular proteins, mRNAs, and such can be used for produced substances. Formation of protrusions and/or change in the number of protrusions, change in flatness, change in the extent of elongation or in the horizontal to vertical ratio, change in cell size, change in internal structure, heteromorphy/homogeneity as a cell population, change in cell density, and such can be used for changes in morphology. Such changes in morphology can be confirmed through microscopic observations. Anchorage dependency, cytokine-dependent responsiveness, hormone dependence, drug resistance, cell motility, cell migration activity, pulsatility, change in intracellular substances, and such can be used for changes in properties. Cell motility includes cell infiltration activity and cell migration activity. Furthermore, for example, enzyme activity, mRNA level, amount of intracellular signaling molecules such as Ca²⁺ and cAMP, intracellular protein level, and such can be used for changes in intracellular substances. In the case of cell membrane receptors, changes in cell proliferation activity induced by receptor stimulation can be used as an indicator.

Functional changes based on the tissues used can be used as a detection indicator for tissue-based systems. Changes in tissue weight, hematologic changes such as change in the number of blood cells, changes in the protein level, enzyme activity, or amount of electrolytes, or changes in the circulatory system such as changes in blood pressure or heart rate can be used as indicators for biological systems.

Methods for measuring these detection indicators are not particularly limited, and absorbance, luminescence, coloring, fluorescence, radioactivity, fluorescence polarization, surface plasmon resonance signal, time-resolved fluorescence, mass, absorption spectrum, light scattering, fluorescence resonance energy transfer, and such can be used. These measurement methods are well known to those skilled in the art, and they can be suitably selected according to the purpose.

For example, absorption spectra can be measured with an ordinarily used photometer, plate reader, or such; luminescence can be measured with a luminometer or such; and fluorescence can be measured with a fluorometer or such. The mass can be measured using a mass spectrometer. Radioactivity can be measured using measuring instruments such as a gamma counter according to the type of radiation; fluorescence polarization can be measured using BEACON (TaKaRa); surface plasmon resonance signals can be measured using BIACORE; time resolved fluorescence, fluorescence resonance energy transfer, and such can be measured using ARVO or such. Flow cytometers and such can also be used for the measurements. Regarding these measurement methods, two or more detection indicators may be measured using one measurement method, and if they are simple, multiple detection indicators can be measured by performing two or more measurements simultaneously and/or sequentially. For example, fluorescence and fluorescence resonance energy transfer can be measured simultaneously on a fluorometer.

In the present invention, measurement of agonistic activity can be performed by methods known to those skilled in the art. For example, as described in the Examples, it is possible to determine by methods that measure the agonistic activity using cell proliferation as an indicator. More specifically, antibodies whose agonistic activity is to be measured are added to cells that show agonist-dependent proliferation and the cells are cultured. Then, the absorbance of a reagent such as WST-8, which exhibits a chromogenic reaction at a particular wavelength depending on the number of live cells added, is measured, and agonistic activity can be measured using the obtained absorbance as an indicator.

Cells showing agonist-dependent proliferation can also be generated by methods known to those skilled in the art, and for example, when the antigen is a receptor emitting cell proliferation signal, cells expressing this receptor can be used. When the antigen is a receptor that does not emit any cell proliferation signal, a chimeric receptor comprising the intracellular region of a receptor emitting cell proliferation signal and the extracellular region of a receptor that does not emit any cell growth signal can be generated, and this chimeric receptor can be expressed in cells. Examples of a receptor that emits cell proliferation signal include the G-CSF receptor, mpl, neu, GM-CSF receptor, EPO receptor, c-kit, and FLT-3. Examples of cells to express the receptors include BaF3, NFS60, FDCP-1, FDCP-2, CTLL-2, DA-1, and KT-3.

The present invention relates to sc(Fv)2 introduced with site-specific mutations.

In the present invention, the sites where the site-specific mutations are introduced are not particularly limited, and may be any site in sc(Fv)2, but preferably, they are any of the following sites:
(a) the 48th amino acid in the heavy chain;
(b) the 65th amino acid in the heavy chain;
(c) the 7th amino acid in the light chain;
(d) the 8th amino acid in the light chain;
(e) the 36th amino acid in the light chain;
(f) the 43rd amino acid in the light chain;
(g) the 45th amino acid in the light chain;
(h) the 70th amino acid in the light chain;
(i) the 81 st amino acid in the heavy chain;
(j) the 39th amino acid in the heavy chain; and
(k) the 38th amino acid in the light chain.

The amino acids after substitution are not particularly limited, and substitution to any amino acid is acceptable, but preferred examples of amino acids after substitution are the following amino acids:
(a) the 48th amino acid in the heavy chain: isoleucine;
(b) the 65th amino acid in the heavy chain: glycine;
(c) the 7th amino acid in the light chain: serine;
(d) the 8th amino acid in the light chain: proline;
(e) the 36th amino acid in the light chain: phenylalanine;
(f) the 43rd amino acid in the light chain: alanine;
(g) the 45th amino acid in the light chain: arginine;
(h) the 70th amino acid in the light chain: aspartic acid;
(i) the 81 st amino acid in the heavy chain: glutamine;
(j) the 39th amino acid in the heavy chain: glutamic acid or lysine; and
(k) the 38th amino acid in the light chain: glutamic acid or lysine.

Therefore, examples of a preferred embodiment of the sc(Fv)2 of the present invention include any of the following sc(Fv)2:
(a) an sc(Fv)2 with the 48th amino acid in the heavy chain substituted;
(b) an sc(Fv)2 with the 65th amino acid in the heavy chain substituted;
(c) an sc(Fv)2 with the 7th amino acid in the light chain is substituted;
(d) an sc(Fv)2 with the 8th amino acid in the light chain substituted;
(e) an sc(Fv)2 with the 36th amino acid in the light chain substituted;
(f) an sc(Fv)2 with the 43rd amino acid in the light chain substituted;
(g) an sc(Fv)2 with the 45th amino acid in the light chain substituted;
(h) an sc(Fv)2 with the 70th amino acid in the light chain substituted;
(i) an sc(Fv)2 with the 81 st amino acid in the heavy chain substituted;
(j) an sc(Fv)2 with the 39th amino acid in the heavy chain substituted; and
(k) an sc(Fv)2 with the 38th amino acid in the light chain substituted.

Moreover, examples of a more preferred embodiment of the present invention include any of the following sc(Fv)2:
(a) an sc(Fv)2 with the 48th amino acid in the heavy chain substituted to isoleucine;
(b) an sc(Fv)2 with the 65th amino acid in the heavy chain substituted to glycine;
(c) an sc(Fv)2 with the 7th amino acid in the light chain substituted to serine;
(d) an sc(Fv)2 with the 8th amino acid in the light chain substituted to proline;
(e) an sc(Fv)2 with the 36th amino acid in the light chain substituted to phenylalanine;
(f) an sc(Fv)2 with the 43rd amino acid in the light chain substituted to alanine;
(g) an sc(Fv)2 with the 45th amino acid in the light chain substituted to arginine;
(h) an sc(Fv)2 with the 70th amino acid in the light chain substituted to aspartic acid;
(i) an sc(Fv)2 with the 81 st amino acid in the heavy chain substituted to glutamine;
(j) an sc(Fv)2 with the 39th amino acid in the heavy chain substituted to glutamic acid or lysine; and
(k) an sc(Fv)2 with the 38th amino acid in the light chain substituted to glutamic acid or lysine.

Furthermore, the present invention relates to sc(Fv)2 in which specific amino acids are positioned at sites that affect the stability of sc(Fv)2. Specifically, the present invention relates to any of the following sc(Fv)2:
(a) an sc(Fv)2 with isoleucine as the 48th amino acid in the heavy chain;
(b) an sc(Fv)2 with glycine as the 65th amino acid in the heavy chain;
(c) an sc(Fv)2 with serine as the 7th amino acid in the light chain;
(d) an sc(Fv)2 with proline as the 8th amino acid in the light chain;
(e) an sc(Fv)2 with phenylalanine as the 36th amino acid in the light chain;
(f) an sc(Fv)2 with alanine as the 43rd amino acid in the light chain;
(g) an sc(Fv)2 with arginine as the 45th amino acid in the light chain;
(h) an sc(Fv)2 with aspartic acid as the 70th amino acid in the light chain;
(i) an sc(Fv)2 with glutamine as the 81 st amino acid in the heavy chain;
(j) an sc(Fv)2 with glutamic acid or lysine as the 39th amino acid in the heavy chain; and
(k) an sc(Fv)2 with glutamic acid or lysine as the 38th amino acid in the light chain.

The invention further relates to sc(Fv)2 that have high Tm values.

In the present invention, a high Tm value refers to a Tm value of 55°C or more, preferably 60°C or more, and more preferably 65°C or more.

Furthermore, the present invention provides sc(Fv)2, whose Tm value has been increased through introduction of site-specific amino acid mutations, by 10°C or more, preferably 13°C or more, and more preferably 15°C or more as compared with the Tm value before introduction of mutations.

The Tm values used in the present invention are Tm values measured under the same conditions as the conditions described in the Examples.

The sc(Fv)2 of the present invention are suitable for use as pharmaceutical compositions because they have excellent properties such as stability and suppressed aggregation. The sc(Fv)2 of the present invention may be any sc(Fv)2, and when they are used as pharmaceutical compositions, without being particularly limited thereto, they are preferably humanized, from the viewpoint of antigenicity against human.

The present invention relates to pharmaceutical compositions comprising an sc(Fv)2 of the present invention. Furthermore, the present invention relates to kits comprising such a pharmaceutical composition and a pharmaceutically acceptable carrier.

The pharmaceutical compositions and kits of the present invention may comprise pharmaceutically acceptable carriers. Examples of pharmaceutically acceptable carriers include sterilized water, physiological saline solution, stabilizers, excipients, antioxidants (such as ascorbic acid), buffers (such as phosphoric acid, citric acid, and other organic acids), antiseptics, surfactants (such as PEG and Tween), chelating agents (such as EDTA), and binders. They may also comprise other low-molecular-weight polypeptides; proteins such as serum albumin, gelatin, and immunoglobulins; amino acids such as glycine, glutamine, asparagine, arginine, and lysine; sugars and carbohydrates such as polysaccharides and monosaccharides; and sugar alcohols such as mannitol and sorbitol. When preparing aqueous solutions for injection, physiological saline solutions, and isotonic solutions comprising glucose or other adjuvants such as D-sorbitol, D-mannose, D-mannitol, and sodium chloride, may be used, and these can be used in combination with suitable solubilizers such as alcohols (for example, ethanol), polyalcohols (such as propylene glycols and PEGs), and non-ionic surfactants (for example, Polysorbate 80 and HCO-50).

If necessary, encapsulation into microcapsules (microcapsules made of hydroxymethylcellulose, gelatin, poly(methylmetacrylate), and such) or preparation into colloidal drug delivery systems (such as liposomes, albumin microspheres, microemulsions, nanoparticles, and nanocapsules) can be carried out (see for example, "Remington's Pharmaceutical Science 16th edition", Oslo Ed. (1980)). Methods for preparing the pharmaceutical agents as sustained-release pharmaceutical agents are also well known, and such methods may be applied to the present invention (Langer et al., J. Biomed. Mater. Res. 1981, 15: 167-277; Langer, Chem. Tech. 1982, 12: 98-105; U.S. Patent No. 3,773,919; European Patent Application Publication (EP) No. 58,481; Sidman et al., Biopolymers 1983, 22: 547-556; EP 133,988).

Administration to patients can be oral or parenteral administration, but is preferably parenteral administration. The form (dosage form) of the pharmaceutical composition of the present invention is not particularly limited, and examples of dosage form include injection, nasal administration, pulmonary administration, transdermal administration, freeze-dried, and solution; and a preferred example is a freeze-dried dosage form.

Freeze drying can be performed by methods well known to those skilled in the art (Pharm. Biotechnol., 2002, 13, 109-33; Int. J. Pharm. 2000, 203(1-2), 1-60; Pharm. Res. 1997, 14(8), 969-75). For example, a suitable amount of a solution is dispensed into a container such as a vial used for freeze-drying, and freeze drying is carried out in a freezer or freeze-dryer, or by immersion in a cooling medium such as acetone/dry ice, liquid nitrogen, or such.

Processes for making antibody formulations into high-concentration solution formulations can be carried out by methods well known to those skilled in the art. For example, as described in a Non-Patent Document (J. Pharm. Sc., 2004, 93(6), 1390-1402), a membrane concentration method using TFF membranes is usually used.

Examples of injection dosage forms include systemic or local administration by intravenous injection, intramuscular injection, intraperitoneal injection, subcutaneous injection, and such. Suitable methods of administration can be selected according to the age and symptoms of the patient. For example, the dosage for each administration can be selected within the range of 0.0001 mg to 1000 mg per kilogram of body weight. Alternatively, for example, the dosage can be selected within the range of 0.001 to 100000 mg/body for each patient. However, the present invention is not limited to these dosages, administration methods, and such.

The present invention relates to methods for producing pharmaceutical compositions comprising sc(Fv)2, which comprise the steps of: (1) introducing site-specific mutations into sc(Fv)2; and (2) mixing with pharmaceutically acceptable carriers.

Examples of pharmaceutically acceptable carriers include those described above.

The numbering of amino acid sites used in the present invention is based on the method by Kabat *et al.* (Kabat EA et al. 1991. Sequence of Proteins of Immunological Interest. NIH).

All prior art references cited herein are incorporated by reference into this description.

### Examples

Hereinafter, the present invention will be specifically described with reference to the Examples, but it is not to be construed as being limited thereto.

### [Example 1] Generation of humanized anti-human Mpl antibody sc(Fv)2

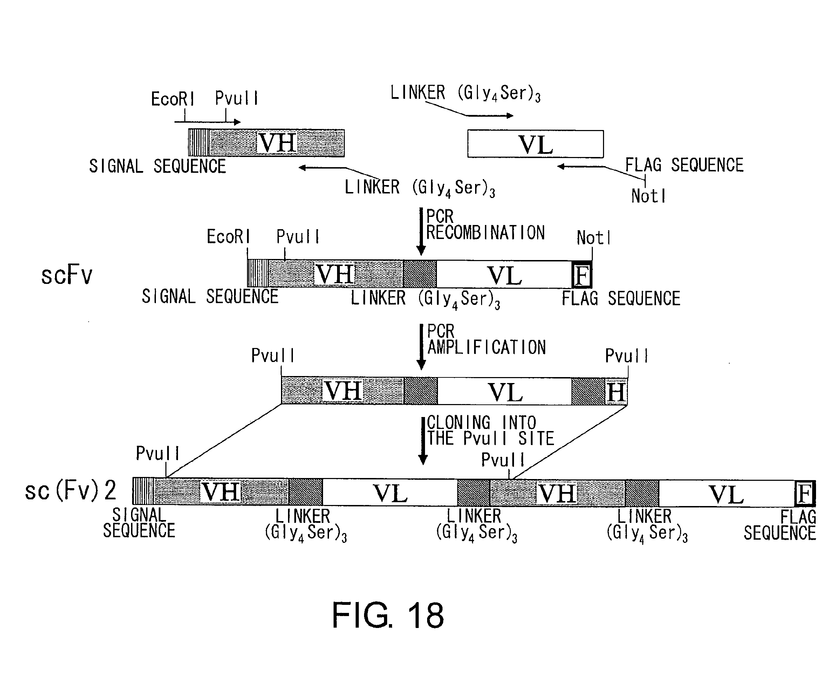

The complementarity determining regions (hereinafter, CDRs) of the mouse anti-human Mpl antibody VB22B were grafted into a highly homologous human antibody framework region (hereinafter, FR) to generate a humanized VB22B variable region gene. Then, the H chain variable region and the L chain variable region were linked through a linker to prepare humanized VB22B sc(Fv)2 by the following method. The process for constructing the humanized VB22B sc(Fv)2 gene is shown in Fig. 18.

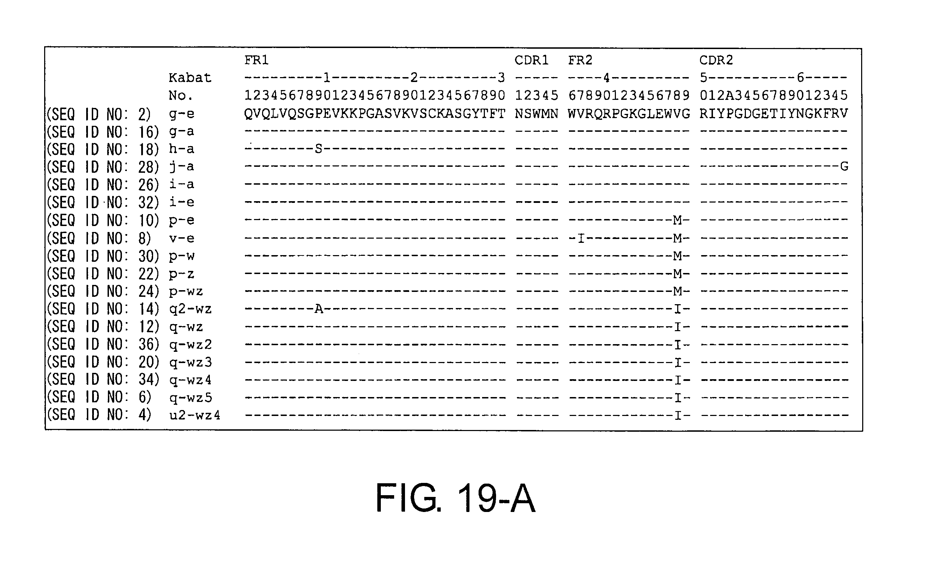

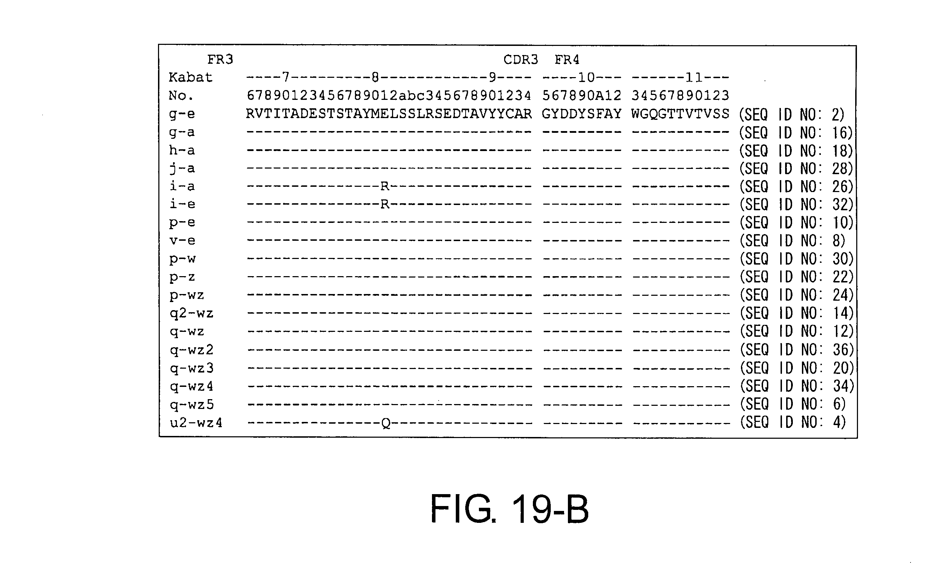

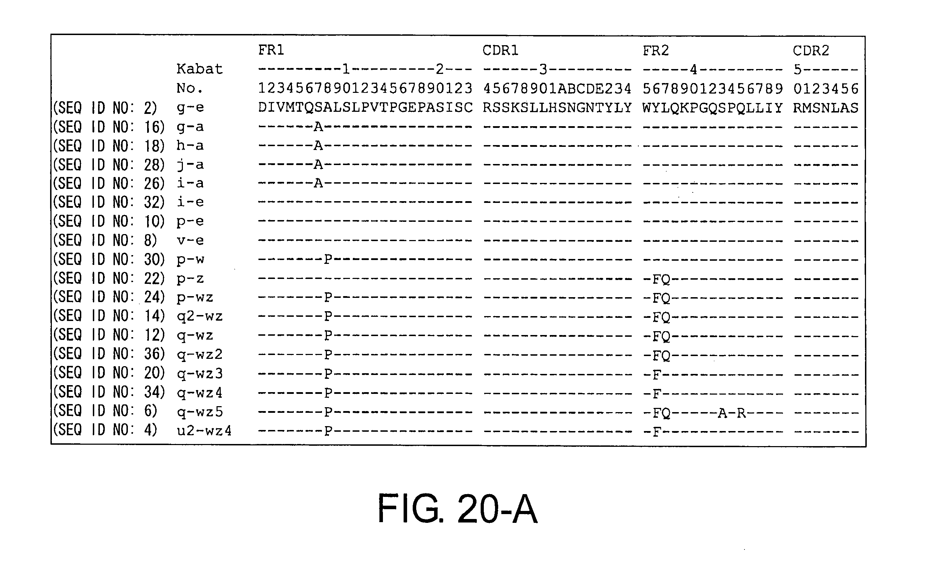

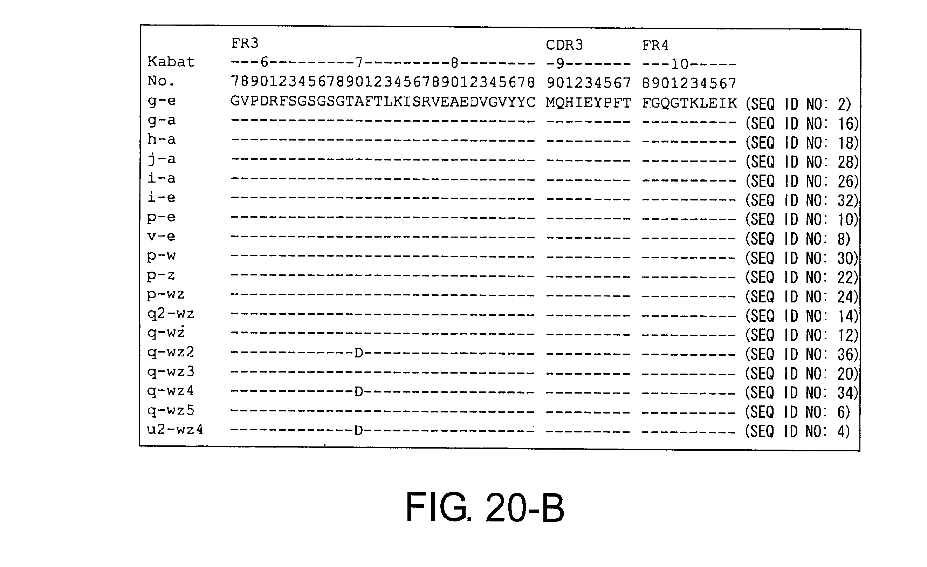

First, genes for the humanized VB22B variable regions were synthesized by assembly PCR. Specifically, synthetic oligo DNAs of about 50 bases were designed so that approximately 20 bases or so would hybridize, and these synthetic oligo DNAs were linked by PCR to prepare genes encoding each of the variable regions. Then, assembly PCR was used to site a nucleotide sequence encoding a linker comprising 15 amino acids (Gly₄Ser)₃ between the 3' end of the gene encoding the humanized VB22B H-chain variable region and the 5' end of the gene encoding the humanized VB22B L-chain variable region. In this construction process, the gene was designed such that the 5' end of the H chain comprises an EcoRI site and the nucleotide sequence encoding the 22nd and 23rd amino acids of the H chain is converted into a PvuII site. Furthermore, the single-chain humanized antibody gene was prepared so that it comprises a nucleotide sequence encoding a NotI site and if necessary, a FLAG sequence at the 3' end of the L chain. Next, a fragment to be inserted into the PvuII site of this single-chain humanized antibody gene was prepared. More specifically, it is a gene encoding a fragment that has a PvuII recognition sequence on both ends, and an N-terminus-deficient H chain variable region linked to the L chain variable region via a (Gly4Ser)3-comprising linker, which is further linked to a gene encoding the N-terminus of the H chain variable region and a nucleotide sequence encoding a (Gly4Ser)3-comprising linker. After digesting this gene fragment with PvuII, this was inserted into the PvuII site of the above-mentioned single-chain humanized antibody gene to produce a humanized antibody sc(Fv)2 gene. Site-specific amino acid mutations were introduced using a QuikChange Site-Directed Mutagenesis Kit (Stratagene) by following the manufacturer's protocol. Each of the completed sc(Fv)2 genes was cloned into the expression vector pCXND3. The VH amino acid sequence and VL amino acid sequence of sc(Fv)2 used in the present description are shown in Fig. 19-A, B and Fig. 20-A, B.

The expression vectors were introduced into CHO-DG44 cells by electroporation, and the cells were added to CHO-S-SFMII medium (Invitrogen) containing 500 µg/mL Geneticin (Invitrogen) and selected to establish CHO expression cell lines. Culture supernatants of these stable expression cell lines were prepared and adsorbed onto an Anti-Flag M2 Affinity Gel (SIGMA-ALDRICH) column equilibrated with 50 mM Tris-HCl (pH 7.4), 150 mM NaCl, and 0.05% Tween20, or in the case of non-Flag tagged sc(Fv)2, onto a column immobilized with the epitope MG10 (a fusion protein with GST of a 19 mer peptide comprising Gln213 to Ala 231 of human Mpl). Then, elution was carried out using 100 mM Glycine-HCl (pH 3.5). The eluted fractions were immediately neutralized with 1 M Tris-HCl (pH 8.0), and subjected-to gel filtration chromatography using a HiLoad 26/60 Superdex 200 pg (Amersham-Bioscience) column.

### [Example 2] Evaluation of the TPO-like agonistic activity of the site-specific mutants of humanized VB22B sc(Fv)2

The TPO-like agonistic activity of hVB22B g-e sc(Fv)2 (the nucleotide sequence is SEQ ID NO: 1, and the amino acid sequence is SEQ ID NO: 2), which is a humanized sc(Fv)2 of anti-Mpl antibody, and those of hVB22B u2-wz4 sc(Fv)2 (the nucleotide sequence is SEQ ID NO: 3, and the amino acid sequence is SEQ ID NO: 4) and hVB22B q-wz5 sc(Fv)2 (the nucleotide sequence is SEQ ID NO: 5, and the amino acid sequence is SEQ ID NO: 6), which are hVB22B g-e sc(Fv)2 into which site-directed mutations have been introduced, were evaluated using BaF-human Mpl cells which show TPO-dependent proliferation. Cells were washed twice with RPMI1640 containing 1% Fetal Bovine Serum (Invitrogen), then suspended at 4x 10⁵ cells/ml in RPMI 1640 containing 10% Fetal Bovine Serum, and this was aliquoted into 96-well plates at 60µl/well. A 40-µL aliquot of rhTPO (R&D) and purified samples prepared at various concentrations was added into each well, and these were incubated at 37°C under 5% CO₂ for 24 hours. WST-8 reagent (Cell Count Reagent SF, Nacalai Tesque) was added at 10-µL/well, and the absorbance at 450 nm (655 nm for the control) was measured using Benchmark Plus immediately after. Absorbance at 450 nm (655 nm for the control) was again measured after two hours of incubation. Since the WST-8 reagent gives a chromogenic reaction at 450 nm in accordance with the viable cell number, TPO-like agonistic activities were evaluated using the change in the absorbance during the two hours as an indicator.

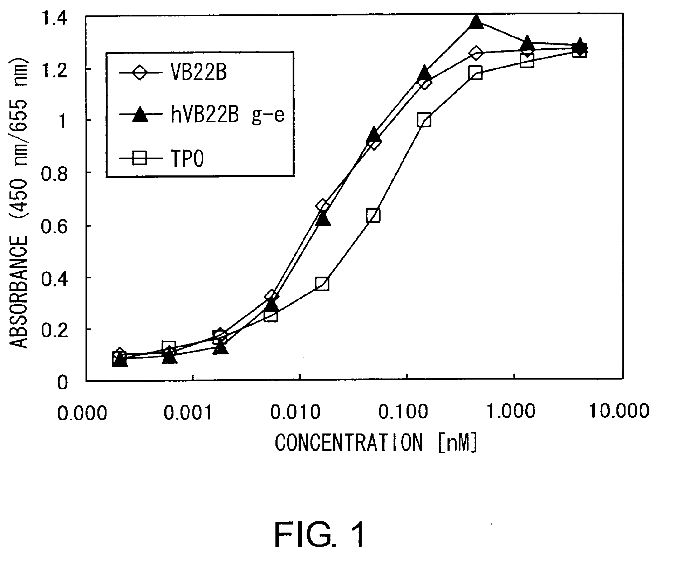

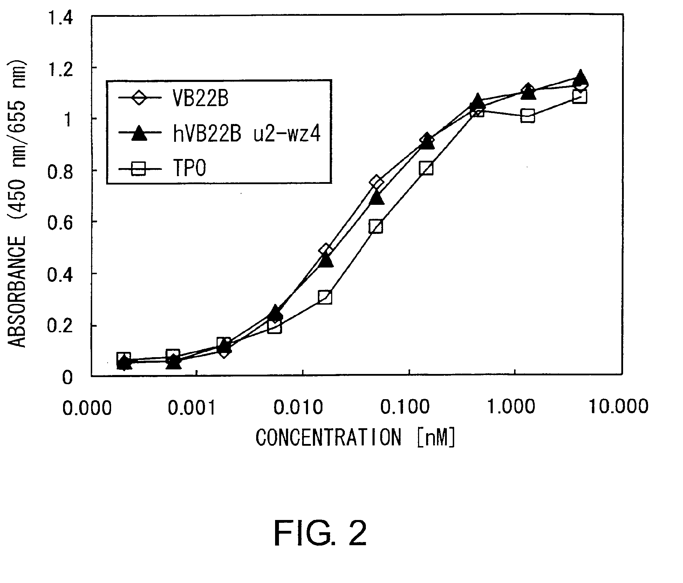

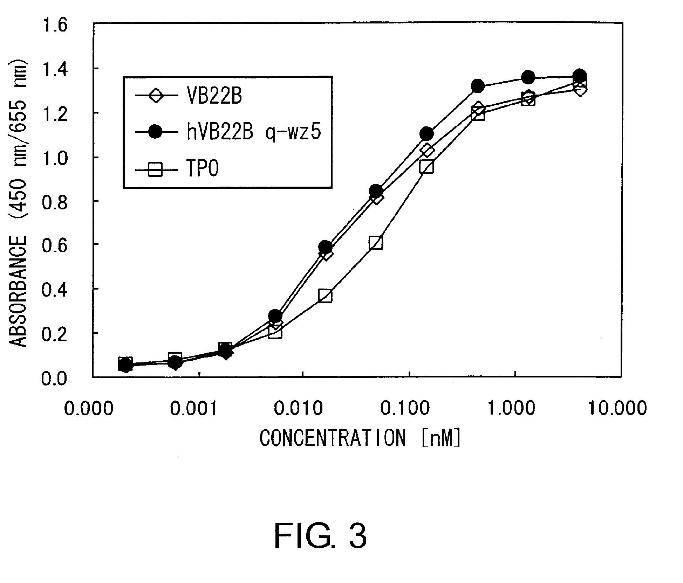

As a result, as shown in Figs. 1, 2, and 3, site-specific mutants of humanized VB22B sc(Fv)2 showed an activity similar to hVB22B g-e sc(Fv)2 before introduction of the mutations and mouse VB22B sc(Fv)2.

### [Example 3] Measurement of Tm values of the site-specific mutants of humanized VB22B sc(Fv)2

Tm values (denaturation midpoint temperatures) were measured using Differential Scanning Calorimetry (DSC) (N-DSC II, Applied Thermodynamics) for hVB22B g-e sc(Fv)2, as well as for hVB22B u2-wz4 sc(Fv)2 and hVB22B q-wz5 sc(Fv)2, which are hVB22B g-e sc(Fv)2 introduced with site-directed mutations. Each sc(Fv)2 was sufficiently dialyzed against 20 mM sodium citrate and 300 mM sodium chloride (pH 7.0), then the concentrations were adjusted to 44.4 µg/mL, denaturation curves were measured using DSC at a scanning speed of 1°C/min, and Tm values were calculated using the attached analytical software.

As a result, DSC curves as those shown in Fig. 4 were obtained, and the Tm values were 53.4°C for hVB22B g-e sc(Fv)2; 66.7°C for hVB22B u2-wz4 sc(Fv)2; and 68.9°C for hVB22B q-wz5 sc(Fv)2. By modifying the amino acids of hVB22B g-e sc(Fv)2 to improve its stability, hVB22B u2-wz4 sc(Fv)2 whose Tm value increased by 13.3°C and hVB22B q-wz5 sc(Fv)2 whose Tm value increased by 15.5°C were obtained. So far there are no reports on the Tm value of sc(Fv)2, or on increasing the Tm value through amino acid modification of sc(Fv)2. As indicated in Example 2, since the agonistic activity was the same before and after amino acid modification, the present inventors succeeded in considerably increasing the Tm value of sc(Fv)2 through amino acid modification without inhibiting antibody function.

### [Example 4] Changes in the stability of sc(Fv)2 through introduction of site-specific mutations

Each sc(Fv)2 was sufficiently dialyzed against 20 mM sodium citrate and 300 mM sodium chloride (pH 7.5), then the concentrations were adjusted to 0.1 mg/mL, and thermal acceleration tests were carried out. The conditions for thermal acceleration are as shown on the horizontal axis of the following Figures. The monomer area was determined by gel filtration chromatography (SEC), and the stability of sc(Fv)2 was evaluated from the change in the ratio of residual monomers over time under each of the thermal acceleration conditions.

The ratio of residual monomers was calculated from "SEC monomer area of the thermal acceleration sample / SEC monomer area of the sample under initial conditions x 100". An increase of the ratio of residual monomers in the thermal acceleration test means improved stability.

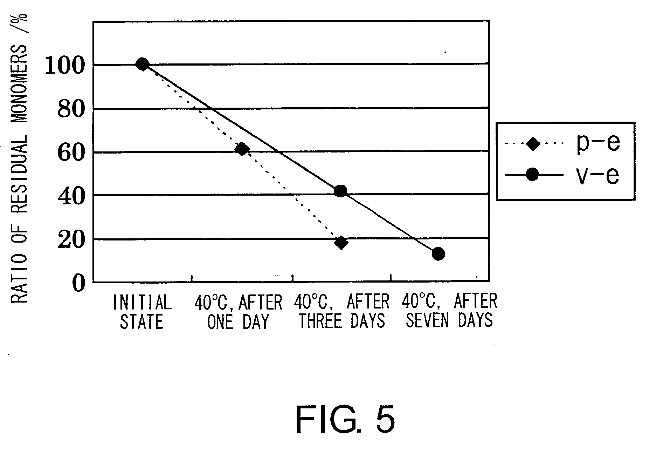

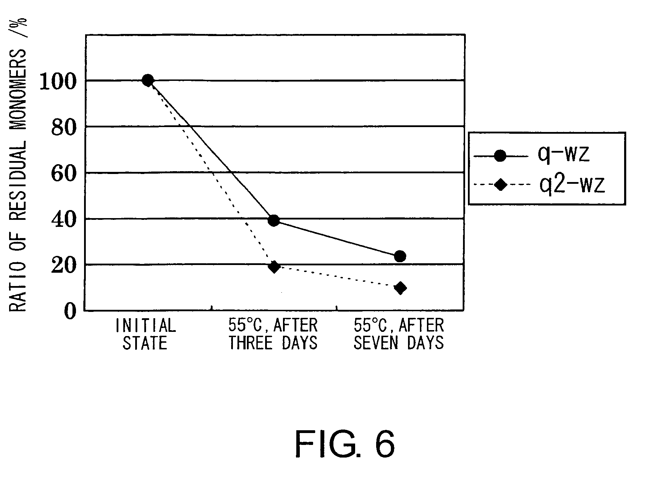

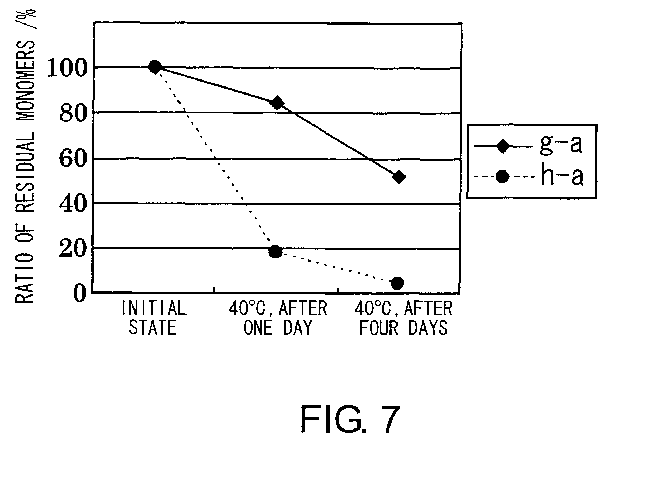

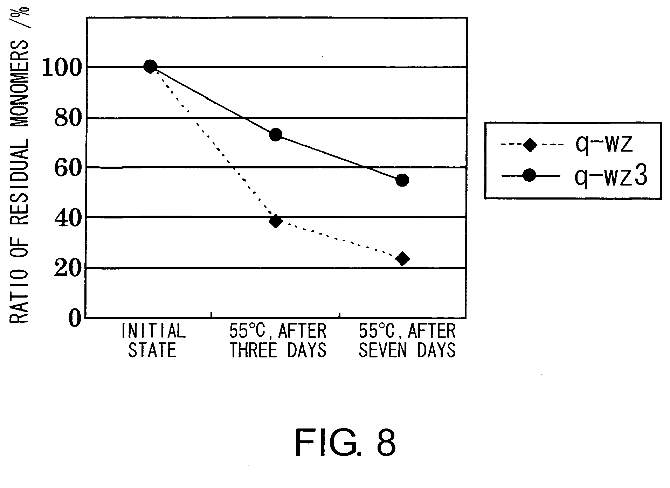

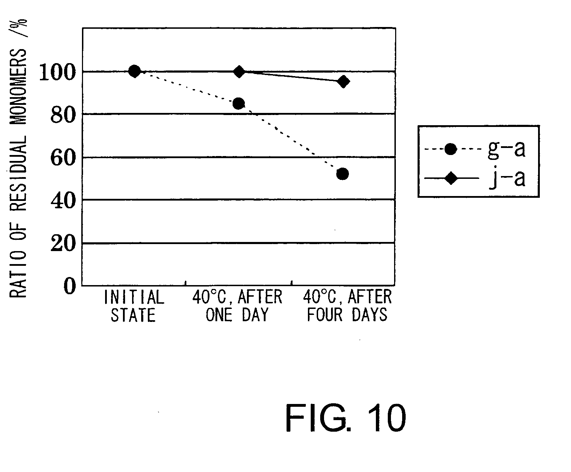

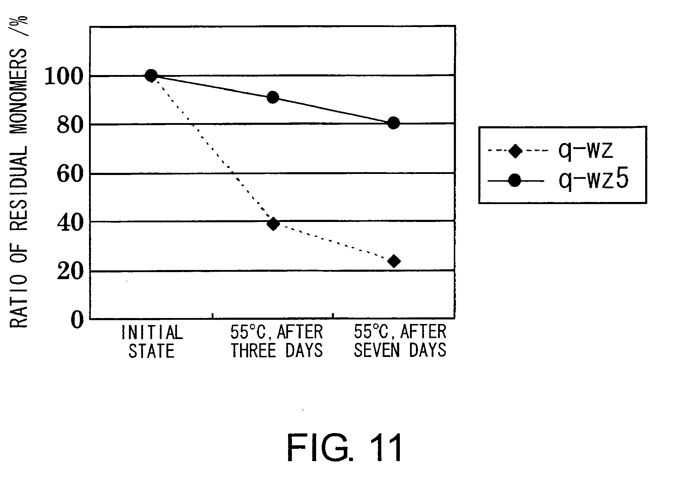

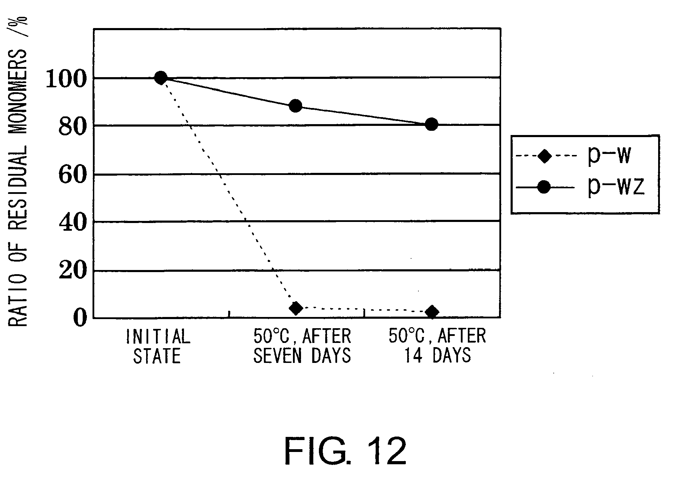

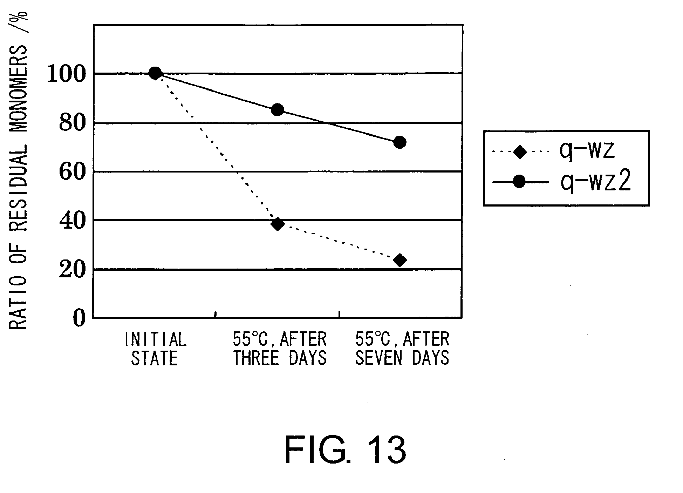

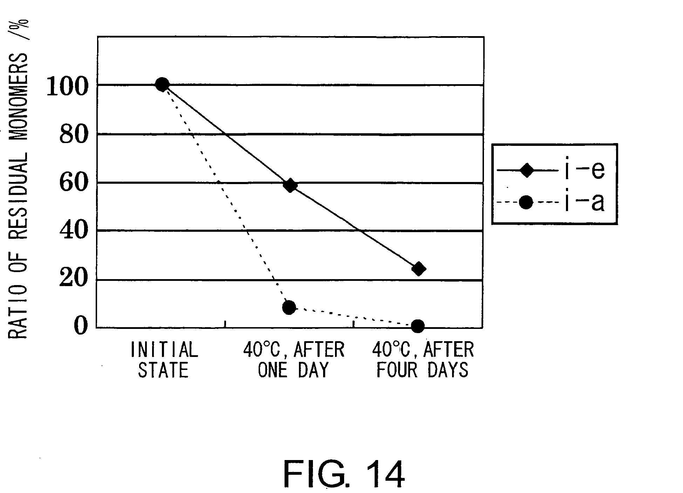

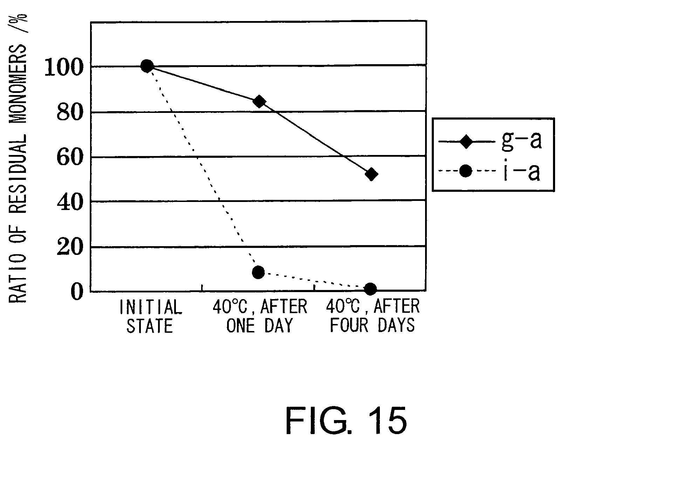

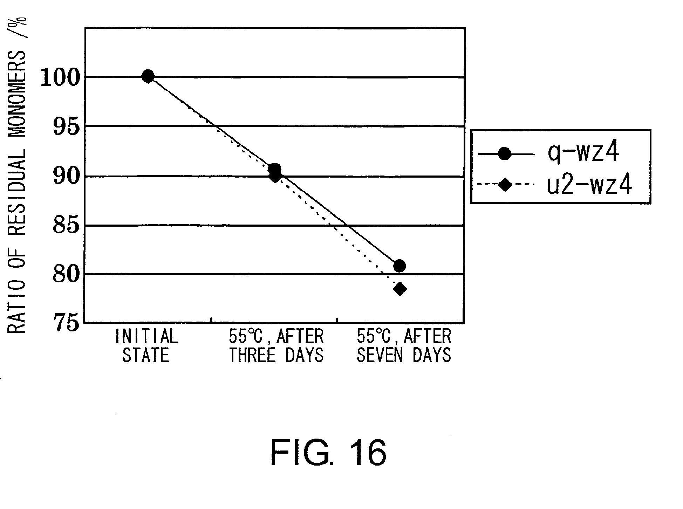

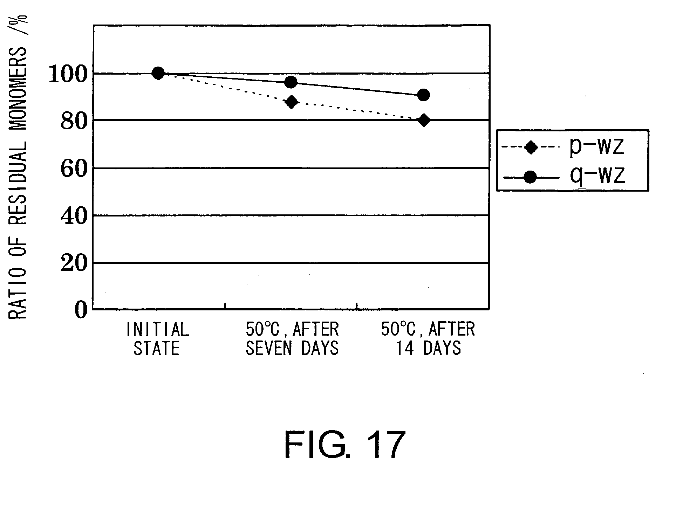

The following amino acid modifications are those reported to have stabilizing effects for scFv, but nevertheless, similar amino acid modifications in sc(Fv)2 did not show any stabilizing effect and instead showed destabilization.
(1) H37 Ile→Val [hVB22B v-e sc(Fv)2 (the nucleotide sequence is SEQ ID NO: 7, and the amino acid sequence is SEQ ID NO: 8) →hVB22B p-e sc(Fv)2 (the nucleotide sequence is SEQ ID NO: 9 and the amino acid sequence is SEQ ID NO: 10), Fig. 5]
   The VH of humanized VB22B is classified into the VH1 subclass and H37 is positioned at the VH/VL interface which plays an important role in stability (J. Mol. Biol. 2001, 305, 989-1010). Since the canonical residue of H37 in the VH1 subclass is Val, modifying H37 from Ile to Val was considered to stabilize the VH/VL interface and improve stability. In fact, in a non-patent document (J. Immunol. Methods, 2003, 275, 31-40), stability is improved by modifying H37 from Met to the canonical residue Val. However, it was revealed that this leads to instead destabilization in sc(Fv)2 (Fig. 5).
(2) H9 Pro→Ala [hVB22B q-wz sc(Fv)2 (the nucleotide sequence is SEQ ID NO: 11, and the amino acid sequence is SEQ ID NO: 12) → hVB22B q2-wz sc(Fv)2 (the nucleotide sequence is SEQ ID NO: 13, and the amino acid sequence is SEQ ID NO: 14), Fig: 6]
   The VH of humanized VB22B is classified into the VH1 subclass, and according to the structure classification described in a non-patent document (J. Mol. Biol. 2001, 309, 687-699), it is classified into type III. The canonical residue of H9 in VH1 is Ala, and according to a non-patent document (J. Mol. Biol. 2001, 309, 701-716), it is known that in all combinations, H9 is more stable as Ala or Gly than as Pro. Therefore, it was thought that stabilization would be accomplished by modifying the H9 of hVB22B q-wz sc(Fv)2 from Pro to Ala which is the canonical residue of type III. However, this was found to instead destabilize sc(Fv)2 (Fig. 6).
(3) H9 Pro→Ser [hVB22B g-a sc(Fv)2 (the nucleotide sequence is SEQ ID NO: 15, and the amino acid sequence is SEQ ID NO: 16) → hVB22B h-a sc(Fv)2 (the nucleotide sequence is SEQ ID NO: 17, and the amino acid sequence is SEQ ID NO: 18), Fig. 7]
   A non-patent document (Protein Eng. 1997, 10(4), 435-444) has reported that in scFv, thermal stability increases by modifying hydrophobic amino acids at the V/C interface to hydrophilic amino acids. Since H9 is positioned at the V/C interface, substitution of the hydrophobic amino acid Pro to the hydrophilic amino acid Ser was thought to lead to stabilization. However, this was found to destabilize sc(Fv)2 instead (Fig. 7).
(4) L37 Leu→Gln [hVB22B q-wz3 sc(Fv)2 (the nucleotide sequence is SEQ ID NO: 19, and the amino acid sequence is SEQ ID NO: 20) → hVB22B q-wz sc(Fv)2 (the nucleotide sequence is SEQ ID NO: 11, and the amino acid sequence is SEQ ID NO: 12), Fig. 8]
   It is indicated in a non-patent document (J. Mol. Biol. 2003, 325, 531-553) that a salt bridge in the VL domain is important for stability, and when L45 is Leu, hydrogen bonds between side chains are not formed and this leads to destabilization. Since L37 of hVB22B q-wz3 sc(Fv)2 is Leu which does not form hydrogen bonds, modification of L37 to Gln was thought to lead to formation of a hydrogen bond network and stabilization. However, this was found to destabilize sc(Fv)2 instead (Fig. 8).
   Next, amino acid modifications that were reported to have stabilizing effects in scFv and also found to have stabilizing effects in sc(Fv)2 are described.
(5) L8 Ala→Pro [hVB22B p-z sc(Fv)2 (the nucleotide sequence is SEQ ID NO: 21, and the amino acid sequence is SEQ ID NO: 22) → hVB22B p-wz sc(Fv)2 (the nucleotide sequence is SEQ ID NO: 23, and the amino acid sequence is SEQ ID NO: 24), Fig. 9]
   L8 is a site in the sequence that has a highly conserved cis-proline structure, and the presence of the cis-proline structure is known to contribute significantly to stability (J. Mol. Biol. 2001, 305, 989-1010). In fact, it is reported in a non-patent document (J. Mol. Biol. 1998, 283, 395-407) that when the L8 of scFv is Pro, this leads to stabilization. Since L8 was Ala in hVB22B p-z sc(Fv)2, when amino acid modification to Pro was performed, a stabilizing effect was observed (Fig. 9).
(6) H65 Val→Gly [hVB22B g-a sc(Fv)2 (the nucleotide sequence is SEQ ID NO: 15, and the amino acid sequence is SEQ ID NO: 16) → hVB22B j-a sc(Fv)2 (the nucleotide sequence is SEQ ID NO: 27, and the amino acid sequence is SEQ ID NO: 28), Fig. 10]
   H65 is known to have a conserved positive φ angle because of the structure of the antibody, and it is reported that H65 is stable as Gly which can form a positive φ angle (J. Mol. Biol. 2001, 305, 989-1010). In fact, it is reported in a non-patent document (Biochemistry, 2003, 42(6), 1517-1528) that making H65 ofscFv from Ser to Gly leads to stabilization. Since H65 of hVB22B g-a sc(Fv)2 was Val, when this was modified to Gly, a stabilizing effect was observed (Fig. 10).
(7) L43 Ser→Ala, L45 Gln→Arg [hVB22B q-wz sc(Fv)2 (the nucleotide sequence is SEQ ID NO: 11, and the amino acid sequence is SEQ ID NO: 12) → hVB22B q-wz5 sc(Fv)2 (the nucleotide sequence is SEQ ID NO: 5, and the amino acid sequence is SEQ ID NO: 6), Fig. 11]
   L45 is positioned at the core stabilized by charge interactions (charge core) present within the antibody, and it has been reported that this charge core influences the stability of scFv (J. Mol. Biol. 2003, 325, 531-553). However, there are no reports that directly showed the influence of two sites, L43 and L45, on stability. Therefore, when L43 and L45 of hVB22B q-wz sc(Fv)2 were modified to Ala and Arg, respectively, a stabilizing effect was observed (Fig. 11).
   Furthermore, amino acid modifications whose stabilization effects have not been reported in scFv but were found to have stabilizing effects in sc(Fv)2 are described.
(8) L36 Tyr→Phe [hVB22B p-w sc(Fv)2 (the nucleotide sequence is SEQ ID NO: 29, and the amino acid sequence is SEQ ID NO: 30) → hVB22B p-wz sc(Fv)2 (the nucleotide sequence is SEQ ID NO: 23, and the amino acid sequence is SEQ ID NO: 24), Fig. 12]
   L36 is positioned at the VH/VL interface, but it is a site for which the influence on stability has not so far been examined even in scFv. In all subclasses, the canonical residue of L36 is Tyr. However, the hydrogen-bond partner for the hydroxyl group of Tyr at L36 is absent, and since hydroxyl groups in the inside which cannot form hydrogen bonds contribute to destabilization, amino acid modification from Tyr to Phe was carried out. Modification to Phe showed a stabilizing effect (Fig. 12).
(9) L70 Ala→Asp [hVB22B q-wz sc(Fv)2 (the nucleotide sequence is SEQ ID NO: 11, and the amino acid sequence is SEQ ID NO: 12) → hVB22B q-wz2 sc(Fv)2 (the nucleotide sequence is SEQ ID NO: 35, and the amino acid sequence is SEQ ID NO: 36), Fig. 13]
   L70 is positioned on the surface of the molecule, but it is a site for which the influence on stability has not so far been examined even in scFv. Modification of L70 from Ala to Asp improved stability.
(10) L7 Ala→Ser [hVB22B i-a-sc(Fv)2 (the nucleotide sequence is SEQ ID NO: 25, and the amino acid sequence is SEQ ID NO: 26) → hVB22B i-e sc(Fv)2 (the nucleotide sequence is SEQ ID NO: 31, and the amino acid sequence is SEQ ID NO: 32), Fig. 14]
   L7 is positioned on the surface of the molecule, but it is a site for which the influence on stability has not so far been examined even in scFv. Modification of L7 from Ala to Ser improved stability.
(11) H81 Gln→Glu [hVB22B i-a sc(Fv)2 (the nucleotide sequence is SEQ ID NO: 25, and the amino acid sequence is SEQ ID NO: 26) → hVB22B g-a sc(Fv)2 (the nucleotide sequence is SEQ ID NO: 15, and the amino acid sequence is SEQ ID NO: 16), Fig. 15] or H81 Arg→Glu [hVB22B u2-wz4 sc(Fv)2 (the nucleotide sequence is SEQ ID NO: 3, and the amino acid sequence is SEQ ID NO: 4) → hVB22B q-wz4 sc(Fv)2 (the nucleotide sequence is SEQ ID NO: 33, and the amino acid sequence is SEQ ID NO: 34), Fig. 16]
   H81 is an amino acid exposed on the surface, and so far, its influence on stability has not been reported even in scFv. It has been reported in a non-patent document (J. Mol. Biol. 2003, 325, 531-553) that the VH3 subclass shows higher stability compared to the VH1 subclass. It has also been reported in a non-patent document (Biochemistry, 2003, 42, 1517-1528) that, in an examination using scFv, stability is improved by modifying the amino acid so that it becomes a canonical residue of the VH3 subclass, and the canonical residue of H81 in the VH3 subclass is Gln. However, in sc(Fv)2, modification of H81 from Gln, which is a canonical residue in the VH3 subclass, to Glu improved stability.
(12) H48 Met→Ile [hVB22B p-wz sc(Fv)2 (the nucleotide sequence is SEQ ID NO: 23, and the amino acid sequence is SEQ ID NO: 24) → hVB22B q-wz sc(Fv)2 (the nucleotide sequence is SEQ ID NO: 11, and the amino acid sequence is SEQ ID NO: 12), Fig. 17]
   There are no reports so far on the influence of H48 on stability even in scFv. The VH1 subclass canonical residue is Met, but stability was improved by modifying H48 from Met to Ile (Fig. 17).

From the above results, it was found that amino acid modifications reported to improve stability in scFv do not necessarily have stabilizing effects in sc(Fv)2 (the mutants of (1)-(4)). This was thought so because the overall three dimensional structures of scFv and sc(Fv)2 are widely different, and the sequence sites that can contribute to stabilization are different in scFv and sc(Fv)2. From these modifications, the present inventors discovered sequence sites that can increase the stability in sc(Fv)2, and stable sequences (the mutants of(5)-(7)). Furthermore, the effects of amino acid modification in sc(Fv)2 at sites for which the influence on stability had not so far been reported in scFv were examined. As a result, sequence sites that improve stability were newly discovered (the mutants of (8)-(12)).

### [Example 5] Generation of sc(Fv)2 with modified VH/VL interface

Gln on site 39 of VH (site 39 in the amino acid sequence of SEQ ID NO: 289 of WO2005/56604) and Gln on site 38 of VL (site 43 in the amino acid sequence of SEQ ID NO: 291 of WO2005/56604), which are amino acids forming the VH/VL interface of hVB22B u2-wz4 sc(Fv)2 (hereinafter, denoted as u2-wz4; the nucleotide sequence is SEQ ID NO: 3, and the amino acid sequence is SEQ ID NO: 4) used in Example 4, were modified as follows. u2-wz4 is linked in the order of [VH1]-linker-[VL2]-linker-[VH3]-linker-[VL4] with an amino acid linker sequence (GlyGlyGlyGlySer)x3 (SEQ ID NO: 37), and is transcribed and translated from the nucleotide sequence of SEQ ID NO: 3. First, the hVB22B u2-wz4(v1) sc(Fv)2 gene (hereinafter denoted as v1; the nucleotide sequence is SEQ ID NO: 38, and the amino acid sequence is SEQ ID NO: 39) was produced with Gln on site 39 of VH1 (genetic codon: CAG) modified to Glu (genetic codon: GAG), Gln on site 38 of VL2 (genetic codon: CAG) modified to Glu (genetic codon: GAG), Gln on site 39 of VH3 (genetic codon: CAG) modified to Lys (genetic codon: AAG), and Gln on site 38 of VL4 (genetic codon: CAG) modified to Lys (genetic codon: AAG). Furthermore, the hVB22B u2-wz4(v3) sc(Fv)2 gene (hereinafter denoted as v3; the nucleotide sequence is SEQ ID NO: 40, and the amino acid sequence is SEQ ID NO: 41) was produced with Gln on site 39 of VH1 (genetic codon: CAG) modified to Glu (genetic codon: GAG), Gln on site 38 of VL2 (genetic codon: CAG) modified to Lys (genetic codon: AAG), Gln on site 39 ofVH3 (genetic codon: CAG) modified to Lys (genetic codon: AAG), and Gln on site 38 of VL4 (genetic codon: CAG) modified to Glu (genetic codon: GAG). Gene modification involved introducing point mutations using a QuikChange Site-Directed Mutagenesis Kit (STRATAGENE) by following the manufacturer's protocol. After confirming the nucleotide sequences of each of the genes, the DNA fragments were cloned into the expression vector pCXND3 to construct expression vectors, and stable expression cell lines were generated by introducing the genes into CHO-DG44 cells. Specifically, a mixture of the expression vector (20 µg) and 0.75 mL of CHO-DG44 cells suspended in PBS (1 x 10⁷ cells/mL) was cooled on ice for ten minutes and transferred to a cuvette, then a pulse was applied at 1.5 kV and a capacitance of 25 µFD using Gene Pulser Xcell (BioRad). After a recovery period of ten minutes at room temperature, cells subjected to electroporation treatment were added into CHO-S-SFMII medium (Invitrogen) containing 500 µg/mL Geneticin (Invitrogen) and selected. A v1-producing CHO cell line and a v3-producing CHO cell line were established.

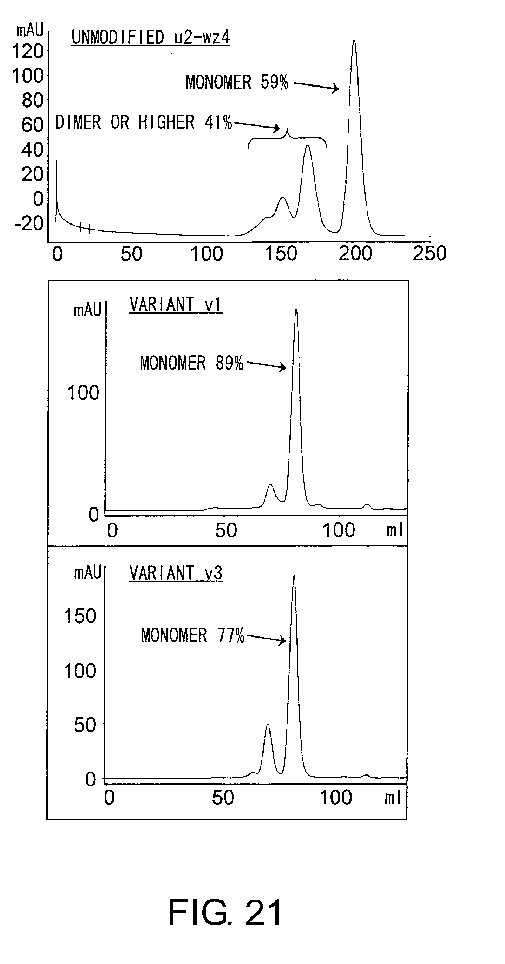

Since the VH/VL interface-modified sc(Fv)2s do not have an added Flag tag, purification from the culture supernatant was carried out using an MG10-GST fusion protein. MG10 (Gln213 to Ala231 of the amino acid sequence of human Mpl) is an epitope recognized by VB22Bsc(Fv)2. The MG10-GST fusion protein was purified using Glutathione Sepharose 4B (Amersham Biosciences) according to the manufacturer's protocol. Further, the purified MG10-GST fusion protein was immobilized onto HiTrap NHS-activated HP (Amersham Biosciences) according to the manufacturer's protocol to prepare an affinity column. The culture supernatant of the v1-expressing CHO cell line or v3-expressing CHO cell line was applied to the MG10-GST fusion protein-immobilized column to adsorb v1 or v3, which were then eluted using 100 mM Glycine-HCl (pH 3.5), 0.01% Tween 80. The eluted fractions were immediately neutralized with 1 M Tris-HCl (pH7.4), and the monomeric molecules were purified by gel filtration chromatography using HiLoad 16/60 Superdex 200 pg (Amersham Biosciences). 20 mM citrate buffer (pH7.5) with 300 mM NaCl and 0.01% Tween 80 was used as a buffer for the gel filtration chromatography. The results of gel filtration chromatography shown in Fig. 21 revealed that dimers and larger aggregates in the culture supernatant decreased for variants v1 and v3, and the proportion of monomers increased from 59% for u2-wz4 before modification to 89% for v1 and 77% for v3. It is speculated that modification of amino acids at the VH/VL interface inhibited unfavorable associations through charge repulsion and promoted favorable association in variants v1 and v3. Accordingly, efficient expression of monomeric molecules was successfully accomplished by this association regulation.

### [Example 6] Evaluation of the stability of VH/VL interface-modified sc(Fv)2

To evaluate the stability of u2-wz4-purified peak 1, u2-wz4-purified peak 2, variant v1, and variant v3, the denaturation midpoint temperature (Tm value) was measured using differential scanning calorimetry under the following conditions:
DSC: N-DSCII (Applied Thermodynamics)
Solution conditions: 20 mM sodium citrate, 300 mM NaCl, pH7.0
Protein concentration: 0.1 mg/mL
Scanning speed: 1°C/minute

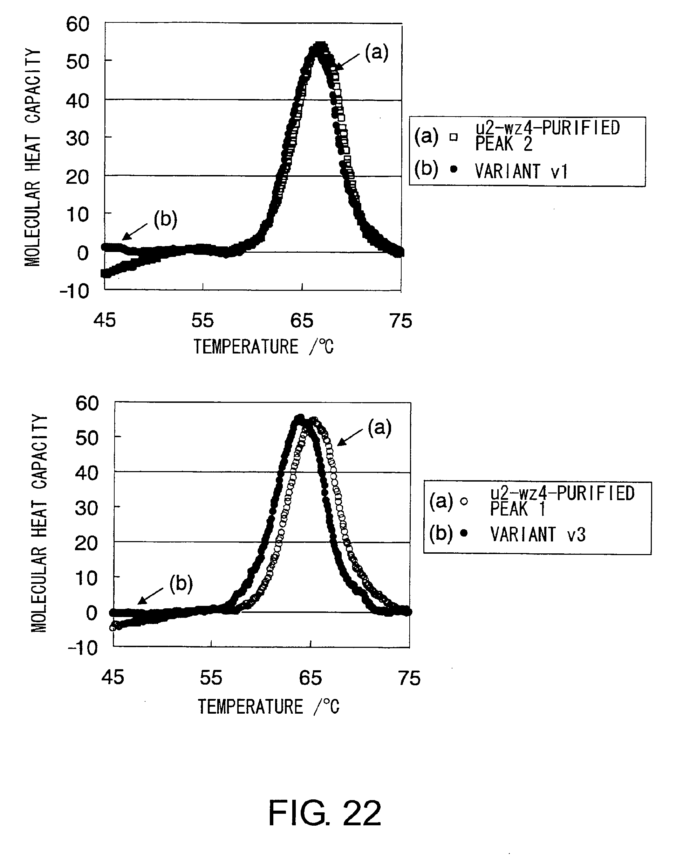

The results of each DSC measurement are shown in Fig. 22. The Tm values for u2-wz4-purified peak 2 and variant v1 were nearly the same as the unmodified form, and their stabilities were found to be the same. Between u2-wz4-purified peak 1 and variant v3, variant v3 showed a slightly lower stability. It has been reported that through regulation of interface by methods using the knobs-into-hole technique, for example, in the heterologous association of IgG CH3 domains, the Tm value for the unmodified CH3 domain was 80.4°C, whereas the Tm value for the modified CH3 domain was 69.4°C, thus the Tm value significantly decreased and stability decreased. In contrast, in the present invention, it was confirmed that aggregation can be regulated without decreasing stability.

Next, stability was evaluated by thermal acceleration tests under the following conditions for u2-wz4-purified peak 1 and u2-wz4-purified peak 2, as well as for the VH/VL interface-modified variants v1 and v3.

### <Thermal acceleration conditions>

Solution conditions: 20 mM sodium citrate, pH 6.0
Protein concentration: 0.25 mg/mL
Acceleration conditions: 40°C - 6 days, 12 days

The thermal acceleration samples were analyzed by gel filtration chromatography and cation exchange chromatography under the following conditions.

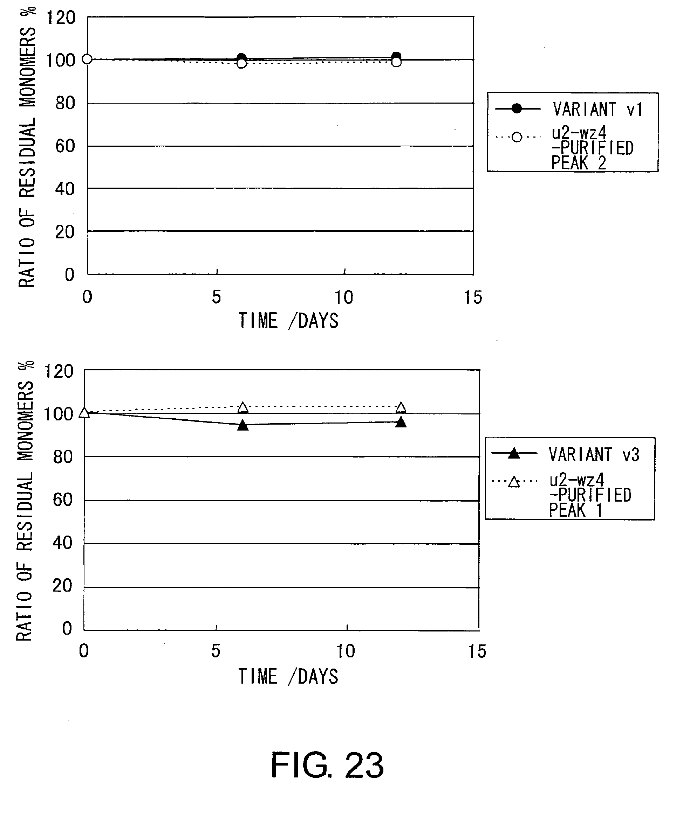

As shown in Fig. 23, the results of gel filtration chromatography analysis confirmed that the ratio of residual monomers is nearly the same for u2-wz4-purified peak 2 and variant v1, and the stability against aggregation was nearly the same. The ratio of residual monomers was also nearly the same for u2-wz4-purified peak 1 and variant v3, and the stability against aggregation was nearly the same for both conformational isomers.

For VH/VL-interface regulation for obtaining a single chain antibody having the conformation of interest, a method which regulates the conformation of bispecific diabodies using the knobs-into-holes technique (Protein Sci. 1997 Apr; 6(4):781-8, Remodeling domain interfaces to enhance heterodimer formation, Zhu Z, Presta LG, Zapata G, Carter P) is known. It was reported that this method increased the percentage of formation of the heterodimeric conformation of interest from 72% to 92% by modifying amino acids at a total of four sites per VH/VL interface. In contrast, the present invention succeeded in obtaining the conformation of interest at a percentage of 100%, without lowering the thermal stability or stability of conformational isomers, by modifying amino acids at four sites.

### Industrial Applicability

By introducing site-specific mutations into sc(Fv)2 or by positioning specific amino acids at specific sites, the aggregation reaction of sc(Fv)2 was suppressed, and it became possible to keep sc(Fv)2 in their monomeric state. To develop antibodies as pharmaceuticals, it is necessary to stably maintain each antibody molecule and to suppress association reactions during storage of formulation to a minimum. Since introduction of the site-specific mutations of the present invention can stabilize sc(Fv)2 in the production storage stage and suppress aggregation reactions, it is considered to be very useful when producing minibody formulations.

In pharmaceutical compositions comprising sc(Fv)2 that are stabilized by the methods of the present invention, since degeneration and association of antibody molecules are suppressed, the decrease in activity due to aggregation is suppressed compared to conventional sc(Fv)2 formulations; thus, these pharmaceutical compositions are expected to maintain potent activity.

## Claims

1. A method for stabilizing an sc(Fv)2, wherein the method comprises the step of introducing a site-specific mutation into the sc(Fv)2.

2. A method for suppressing association between sc(Fv)2s, wherein the method comprises the step of introducing a site-specific mutation into the sc(Fv)2s.

3. A method for increasing the Tm value of an sc(Fv)2 by 10°C or more, wherein the method comprises the step of introducing a site-specific mutation into the sc(Fv)2.

4. The method of any one of claims 1 to 3, wherein the introduction of a site-specific mutation introduces a mutation to at least one amino acid selected from:
(a) the 48th amino acid in the heavy chain;
(b) the 65th amino acid in the heavy chain;
(c) the 7th amino acid in the light chain;
(d) the 8th amino acid in the light chain;
(e) the 36th amino acid in the light chain;
(f) the 43rd amino acid in the light chain;
(g) the 45th amino acid in the light chain;
(h) the 70th amino acid in the light chain;
(i) the 81 st amino acid in the heavy chain;
(j) the 39th amino acid in the heavy chain; and
(k) the 38th amino acid in the light chain.

5. The method of any one of claims 1 to 4, wherein the introduction of a site-specific mutation introduces at least one amino acid mutation selected from:
(a) substitution of the 48th amino acid in the heavy chain to isoleucine;
(b) substitution of the 65th amino acid in the heavy chain to glycine;
(c) substitution of the 7th amino acid in the light chain to serine;
(d) substitution of the 8th amino acid in the light chain to proline;
(e) substitution of the 36th amino acid in the light chain to phenylalanine;
(f) substitution of the 43rd amino acid in the light chain to alanine;
(g) substitution of the 45th amino acid in the light chain to arginine;
(h) substitution of the 70th amino acid in the light chain to aspartic acid;
(i) substitution of the 81 st amino acid in the heavy chain to glutamine;
(j) substitution of the 39th amino acid in the heavy chain to glutamic acid or lysine; and
(k) substitution of the 38th amino acid in the light chain to glutamic acid or lysine.

6. A method for stabilizing an sc(Fv)2 by any one of the following methods:
(a) a method for converting the 48th amino acid in the heavy chain to isoleucine;
(b) a method for converting the 65th amino acid in the heavy chain to glycine;
(c) a method for converting the 7th amino acid in the light chain to serine;
(d) a method for converting the 8th amino acid in the light chain to proline;
(e) a method for converting the 36th amino acid in the light chain to phenylalanine;
(f) a method for converting the 43rd amino acid in the light chain to alanine;
(g) a method for converting the 45th amino acid in the light chain to arginine;
(h) a method for converting the 70th amino acid in the light chain to aspartic acid;
(i) a method for converting the 81 st amino acid in the heavy chain to glutamine;
(j) a method for converting the 39th amino acid in the heavy chain to glutamic acid or lysine; and
(k) a method for converting the 38th amino acid in the light chain to glutamic acid or lysine.

7. An sc(Fv)2 into which a mutation has been introduced to at least one amino acid selected from:
(a) the 48th amino acid in the heavy chain;
(b) the 65th amino acid in the heavy chain;
(c) the 7th amino acid in the light chain;
(d) the 8th amino acid in the light chain;
(e) the 36th amino acid in the light chain;
(f) the 43rd amino acid in the light chain;
(g) the 45th amino acid in the light chain;
(h) the 70th amino acid in the light chain;
(i) the 81 st amino acid in the heavy chain;
(j) the 39th amino acid in the heavy chain; and
(k) the 38th amino acid in the light chain.

8. An sc(Fv)2 into which at least one amino acid mutation selected from the following (a) to (k) has been introduced:
(a) substitution of the 48th amino acid in the heavy chain to isoleucine;
(b) substitution of the 65th amino acid in the heavy chain to glycine;
(c) substitution of the 7th amino acid in the light chain to serine;
(d) substitution of the 8th amino acid in the light chain to proline;
(e) substitution of the 36th amino acid in the light chain to phenylalanine;
(f) substitution of the 43rd amino acid in the light chain to alanine;
(g) substitution of the 45th amino acid in the light chain to arginine;
(h) substitution of the 70th amino acid in the light chain to aspartic acid;
(i) substitution of the 81 st amino acid in the heavy chain to glutamine;
(j) substitution of the 39th amino acid in the heavy chain to glutamic acid or lysine; and
(k) substitution of the 38th amino acid in the light chain to glutamic acid or lysine.

9. An sc(Fv)2 selected from:
(a) an sc(Fv)2 with isoleucine as the 48th amino acid in the heavy chain;
(b) an sc(Fv)2 with glycine as the 65th amino acid in the heavy chain;
(c) an sc(Fv)2 with serine as the 7th amino acid in the light chain;
(d) an sc(Fv)2 with proline as the 8th amino acid in the light chain;
(e) an sc(Fv)2 with phenylalanine as the 36th amino acid in the light chain;
(f) an sc(Fv)2 with alanine as the 43rd amino acid in the light chain;
(g) an sc(Fv)2 with arginine as the 45th amino acid in the light chain;
(h) an sc(Fv)2 with aspartic acid as the 70th amino acid in the light chain;
(i) an sc(Fv)2 with glutamine as the 81 st amino acid in the heavy chain;
(j) an sc(Fv)2 with glutamic acid or lysine as the 39th amino acid in the heavy chain; and
(k) an sc(Fv)2 with glutamic acid or lysine as the 38th amino acid in the light chain.

10. An sc(Fv)2 whose Tm value is 55°C or higher.

11. An sc(Fv)2 whose Tm value has increased by 10°C or more by the introduction of a site-specific amino acid mutation, as compared with before the introduction.

12. A pharmaceutical composition comprising the sc(Fv)2 of any one of claims 7 to 11.

13. A method for producing the pharmaceutical composition of claim 12, wherein the method comprises the steps of:
(1) introducing the site-specific mutation of any one of claims 1 to 5 into the sc(Fv)2; and
(2) mixing with a pharmaceutically acceptable carrier.


SEQUENCE LISTING

<110> CHUGAI SEIYAKU KABUSHIKI KAISHA

<120> sc(Fv)2 SITE-DIRECTED MUTANT

<130> C1-A0510Y1P

<150> JP 2005-171673
<151> 2005-06-10

<150> JP 2005-378639
<151> 2005-12-28

<160> 49

<170> PatentIn version 3.3

<210> 1
<211> 1572
<212> DNA
<213> Artificial

<220>
<223> an artificially synthesized nucleotide sequence

<400> 1
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtgggt tggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctgca 480

ctctccctgc ccgtcacccc tggagagccg gcctccatct cctgcaggtc tagtaagagt 540

ctcctgcata gtaatggcaa cacttacttg tattggtacc tgcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtgggt tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 2
<211> 524
<212> PRT
<213> Artificial

<220>
<223> an artificially synthesized peptide sequence

<400> 2
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Val Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Val Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  3
<211>  1572
<212>  DNA
<213>  Artificial

<220>
<223>  an artificially synthesized nucleotide sequence

<400>  3
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actgggtgag gcagaggcct     180

ggaaagggtc ttgagtggat tggacggatt tatcctggag atggagaaac tatctacaat     240

gggaaattca gggtcagagt cacgattacc gcggacgaat ccacgagcac agcctacatg     300

caactgagca gcctgagatc tgaggacacg gccgtgtatt actgtgcgag aggctatgat     360

gattactcgt ttgcttactg gggccaggga accacggtca ccgtctcttc aggtggtggt     420

ggatccggag gtggtggatc gggtggtgga ggatcggata ttgtgatgac tcagtctcca     480

ctctccctgc ccgtcacccc tggagagccg gcctccatct cctgcaggtc tagtaagagt 540

ctcctgcata gtaatggcaa cacttacttg tattggttcc tgcagaagcc agggcagtct 600

ccacagctcc tgatctatcg gatgtccaac cttgcctcag gggtccctga caggttcagt 660

ggcagtggat caggcacaga ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

caactgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc tgcagaagcc agggcagtct 1380

ccacagctcc tgatctatcg gatgtccaac cttgcctcag gggtccctga caggttcagt 1440

ggcagtggat caggcacaga ttttacactg aaaatcagca gagtggaggc tgaggatgtt 1500

ggggtttatt actgcatgca acatatagaa tatcctttta cgttcggcca agggaccaaa 1560

ctggaaatca aa                                                          1572

<210>  4
<211>  524
<212>  PRT
<213>  Artificial

<220>
<223>  an artificially synthesized peptide sequence

<400>  4
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Gln Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Asp Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Ile Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Gln Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Asp Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  5
<211>  1572
<212>  DNA
<213>  Artificial

<220>
<223>  an artificially synthesized nucleotide sequence

<400>  5
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actgggtgag gcagaggcct     180

ggaaagggtc ttgagtggat tggacggatt tatcctggag atggagaaac tatctacaat     240

gggaaattca gggtcagagt cacgattacc gcggacgaat ccacgagcac agcctacatg     300

gagctgagca gcctgagatc tgaggacacg gccgtgtatt actgtgcgag aggctatgat     360

gattactcgt ttgcttactg gggccaggga accacggtca ccgtctcttc aggtggtggt     420

ggatccggag gtggtggatc gggtggtgga ggatcggata ttgtgatgac tcagtctcca     480

ctctccctgc ccgtcacccc tggagagccg gcctccatct cctgcaggtc tagtaagagt     540

ctcctgcata gtaatggcaa cacttacttg tattggttcc agcagaagcc agggcaggct     600

ccacggctcc tgatctatcg gatgtccaac cttgcctcag gggtccctga caggttcagt     660

ggcagtggat caggcacagc ttttacactg aaaatcagca gagtggaggc tgaggatgtt     720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc agcagaagcc agggcaggct 1380

ccacggctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 6
<211> 524
<212> PRT
<213> Artificial

<220>

<223>  an artificially synthesized peptide sequence

<400>  6

Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg

                165                 170                 175

Ser Ser Lys Ser Leu Leu His Ser Asn Gly Asn Thr Tyr Leu Tyr Trp
            180                 185                 190

Phe Gln Gln Lys Pro Gly Gln Ala Pro Arg Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Ile Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu

        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Gln Gln Lys Pro Gly Gln Ala Pro Arg Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210> 7
<211> 1572
<212> DNA

<213>  Artificial

<220>
<223>  an artificially synthesized nucleotide sequence

<400>  7
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actggatcag gcagaggcct     180

ggaaagggtc ttgagtggat gggacggatt tatcctggag atggagaaac tatctacaat     240

gggaaattca gggtcagagt cacgattacc gcggacgaat ccacgagcac agcctacatg     300

gagctgagca gcctgagatc tgaggacacg gccgtgtatt actgtgcgag aggctatgat     360

gattactcgt ttgcttactg gggccaggga accacggtca ccgtctcttc aggtggtggt     420

ggatccggag gtggtggatc gggtggtgga ggatcggata ttgtgatgac tcagtctgca     480

ctctccctgc ccgtcacccc tggagagccg gcctccatct cctgcaggtc tagtaagagt     540

ctcctgcata gtaatggcaa cacttacttg tattggtacc tgcagaagcc agggcagtct     600

ccacagctcc tgatctatcg gatgtccaac cttgcctcag gggtccctga caggttcagt     660

ggcagtggat caggcacagc ttttacactg aaaatcagca gagtggaggc tgaggatgtt     720

ggggtttatt actgcatgca acatatagaa tatcctttta cgttcggcca agggaccaaa     780

ctggaaatca aaggaggtgg tggatcgggt ggtggtggtt cgggaggcgg tggatcgcag     840

gtgcagctgg tgcagtctgg acctgaggtg aagaagcctg gggcctcagt gaaggtctcc     900

tgcaaggctt ctggatacac cttcaccaac tcctggatga actggatcag gcagaggcct 960

ggaaagggtc ttgagtggat gggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 8
<211> 524
<212> PRT
<213> Artificial

<220>
<223> an artificially synthesized peptide sequence

<400> 8
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Ile Arg Gln Arg Pro Gly Lys Gly Leu
    50                  55                  60

Glu Trp Met Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Ile Arg Gln Arg Pro
305                 310                 315                 320

Gly Lys Gly Leu Glu Trp Met Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  9
<211>  1572
<212>  DNA
<213>  Artificial

<220>
<223>  an artificially synthesized nucleotide sequence

<400>  9
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtggat gggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctgca 480

ctctccctgc ccgtcacccc tggagagccg gcctccatct cctgcaggtc tagtaagagt 540

ctcctgcata gtaatggcaa cacttacttg tattggtacc tgcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat gggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 10
<211> 524
<212> PRT
<213> Artificial

<220>
<223> an artificially synthesized peptide sequence

<400> 10
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
50                  55                  60

Glu Trp Met Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Met Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  11
<211>  1572
<212>  DNA
<213>  Artificial

<220>
<223>  an artificially synthesized nucleotide sequence

<400>  11
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actgggtgag gcagaggcct     180

ggaaagggtc ttgagtggat tggacggatt tatcctggag atggagaaac tatctacaat     240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctcca 480

ctctccctgc ccgtcacccc tggagagccg gcctccatct cctgcaggtc tagtaagagt 540

ctcctgcata gtaatggcaa cacttacttg tattggttcc agcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc agcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 12
<211> 524
<212> PRT
<213> Artificial

<220>
<223> an artificially synthesized peptide sequence

<400> 12
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser

                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Gln Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly.Gly Gly Gly Ser Gln Val Gln Leu Val Gln Ser Gly Pro

275 280 285

Glu Val Lys Lys Pro Gly Ala Ser Val Lys Val Ser Cys Lys Ala Ser
290 295 300

Gly Tyr Thr Phe Thr Asn Ser Trp Met Asn Trp Val Arg Gln Arg Pro
305 310 315 320

Gly Lys Gly Leu Glu Trp Ile Gly Arg Ile Tyr Pro Gly Asp Gly Glu
325 330 335

Thr Ile Tyr Asn Gly Lys Phe Arg Val Arg Val Thr Ile Thr Ala Asp
340 345 350

Glu Ser Thr Ser Thr Ala Tyr Met Glu Leu Ser Ser Leu Arg Ser Glu
355 360 365

Asp Thr Ala Val Tyr Tyr Cys Ala Arg Gly Tyr Asp Asp Tyr Ser Phe
370 375 380

Ala Tyr Trp Gly Gln Gly Thr Thr Val Thr Val Ser Ser Gly Gly Gly
385 390 395 400

Gly Ser Gly Gly Gly Gly Ser Gly Gly Gly Gly Ser Asp Ile Val Met
405 410 415

Thr Gln Ser Pro Leu Ser Leu Pro Val Thr Pro Gly Glu Pro Ala Ser
420 425 430

Ile Ser Cys Arg Ser Ser Lys Ser Leu Leu His Ser Asn Gly Asn Thr
435 440 445

Tyr Leu Tyr Trp Phe Gln Gln Lys Pro Gly Gln Ser Pro Gln Leu Leu
450 455 460

Ile Tyr Arg Met Ser Asn Leu Ala Ser Gly Val Pro Asp Arg Phe Ser

465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  13
<211>  1572
<212>  DNA
<213>  Artificial

<220>
<223>  an artificially synthesized nucleotide sequence

<400>  13
atggactgga cctggaggtt cctctttgtg gtggcagcag ctacaggtgt ccagtcccag      60

gtgcagctgg tgcagtctgg agctgaggtg aagaagcctg gggcctcagt gaaggtctcc     120

tgcaaggctt ctggatacac cttcaccaac tcctggatga actgggtgag gcagaggcct     180

ggaaagggtc ttgagtggat tggacggatt tatcctggag atggagaaac tatctacaat     240

gggaaattca gggtcagagt cacgattacc gcggacgaat ccacgagcac agcctacatg     300

gagctgagca gcctgagatc tgaggacacg gccgtgtatt actgtgcgag aggctatgat     360

gattactcgt ttgcttactg gggccaggga accacggtca ccgtctcttc aggtggtggt     420

ggatccggag gtggtggatc gggtggtgga ggatcggata ttgtgatgac tcagtctcca     480

ctctccctgc ccgtcacccc tggagagccg gcctccatct cctgcaggtc tagtaagagt 540

ctcctgcata gtaatggcaa cacttacttg tattggttcc agcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg agctgaggtg aagaagcctg gggcctcagt gaaggtctcc 900

tgcaaggctt ctggatacac cttcaccaac tcctggatga actgggtgag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc agcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa                                                          1572

<210>  14
<211>  524
<212>  PRT
<213>  Artificial

<220>
<223>  an artificially synthesized peptide sequence

<400>  14
Met Asp Trp Thr Trp Arg Phe Leu Phe Val Val Ala Ala Ala Thr Gly
1               5                   10                  15

Val Gln Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Gln Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Ala
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Ile Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Gln Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210> 15
<211> 1572
<212> DNA
<213> Artificial

<220>
<223> an artificially synthesized nucleotide sequence

<400> 15
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtgggt tggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcaggctgca 480

ctctccctgc ccgtcacccc tggagagccg gcctccatct cctgcaggtc tagtaagagt 540

ctcctgcata gtaatggcaa cacttacttg tattggtacc tgcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtgggt tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcaggctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 16
<211> 524
<212> PRT

<213>  Artificial

<220>
<223>  an artificially synthesized peptide sequence

<400>  16
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Val Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ala Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Val Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ala Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210> 17
<211> 1572

<212>  DNA
<213>  Artificial

<220>
<223>  an artificially synthesized nucleotide sequence

<400>  17
atggactgga cctggaggtt cctctttgtg gtggcagcag ctacaggtgt ccagtcccag      60

gtgcagctgg tgcagtctgg atctgaggtg aagaagcctg gggcctcagt gaaggtctcc     120

tgcaaggctt ctggatacac cttcaccaac tcctggatga actgggtgag gcagaggcct     180

ggaaagggtc ttgagtgggt tggacggatt tatcctggag atggagaaac tatctacaat     240

gggaaattca gggtcagagt cacgattacc gcggacgaat ccacgagcac agcctacatg     300

gagctgagca gcctgagatc tgaggacacg gccgtgtatt actgtgcgag aggctatgat     360

gattactcgt ttgcttactg gggccaggga accacggtca ccgtctcttc aggtggtggt     420

ggatccggag gtggtggatc gggtggtgga ggatcggata ttgtgatgac tcaggctgca     480

ctctccctgc ccgtcacccc tggagagccg gcctccatct cctgcaggtc tagtaagagt     540

ctcctgcata gtaatggcaa cacttacttg tattggtacc tgcagaagcc agggcagtct     600

ccacagctcc tgatctatcg gatgtccaac cttgcctcag gggtccctga caggttcagt     660

ggcagtggat caggcacagc ttttacactg aaaatcagca gagtggaggc tgaggatgtt     720

ggggtttatt actgcatgca acatatagaa tatcctttta cgttcggcca agggaccaaa     780

ctggaaatca aaggaggtgg tggatcgggt ggtggtggtt cgggaggcgg tggatcgcag     840

gtgcagctgg tgcagtctgg atctgaggtg aagaagcctg gggcctcagt gaaggtctcc     900

tgcaaggctt ctggatacac cttcaccaac tcctggatga actgggtgag gcagaggcct 960

ggaaagggtc ttgagtgggt tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcaggctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 18
<211> 524
<212> PRT
<213> Artificial

<220>
<223> an artificially synthesized peptide sequence

<400> 18
Met Asp Trp Thr Trp Arg Phe Leu Phe Val Val Ala Ala Ala Thr Gly

1               5                   10                  15

Val Gln Ser Gln Val Gln Leu Val Gln Ser Gly Ser Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Val Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ala Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met

        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Ser
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Val Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly

385                 390                 395                 400

Gly Ser Gly Gly Gly Gly Ser Gly Gly Gly Gly Ser Asp Ile Val Met
                405                 410                 415

Thr Gln Ala Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  19
<211>  1572
<212>  DNA
<213>  Artificial

<220>
<223>  an artificially synthesized nucleotide sequence

<400>  19

atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtggat tggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctcca 480

ctctccctgc ccgtcacccc tggagagccg gcctccatct cctgcaggtc tagtaagagt 540

ctcctgcata gtaatggcaa cacttacttg tattggttcc tgcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat    1140

gattactcgt ttgcttactg gggccaggga accacggtca ccgtctcttc aggtggtggt    1200

ggatccggag gtggtggatc gggtggtgga ggatcggata ttgtgatgac tcagtctcca    1260

ctctccctgc ccgtcacccc tggagagccg gcctccatct cctgcaggtc tagtaagagt    1320

ctcctgcata gtaatggcaa cacttacttg tattggttcc tgcagaagcc agggcagtct    1380

ccacagctcc tgatctatcg gatgtccaac cttgcctcag gggtccctga caggttcagt    1440

ggcagtggat caggcacagc ttttacactg aaaatcagca gagtggaggc tgaggatgtt    1500

ggggtttatt actgcatgca acatatagaa tatcctttta cgttcggcca agggaccaaa    1560

ctggaaatca aa                                                        1572

<210>  20
<211>  524
<212>  PRT
<213>  Artificial

<220>
<223>  an artificially synthesized peptide sequence

<400>  20
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Ile Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210> 21
<211> 1572
<212> DNA
<213> Artificial

<220>
<223> an artificially synthesized nucleotide sequence

<400> 21
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actgggtgag gcagaggcct     180

ggaaagggtc ttgagtggat gggacggatt tatcctggag atggagaaac tatctacaat     240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctgca 480

ctctccctgc ccgtcacccc tggagagccg gcctccatct cctgcaggtc tagtaagagt 540

ctcctgcata gtaatggcaa cacttacttg tattggttcc agcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat gggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctgca 1260

ctctccctgc ccgtcacccc tggagagccg gcctccatct cctgcaggtc tagtaagagt 1320

ctcctgcata gtaatggcaa cacttacttg tattggttcc agcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 22
<211> 524
<212> PRT
<213> Artificial

<220>
<223> an artificially synthesized peptide sequence

<400> 22
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Met Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Gln Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Met Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Gln Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  23
<211>  1572
<212>  DNA
<213>  Artificial

<220>
<223>  an artificially synthesized nucleotide sequence

<400>  23
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actgggtgag gcagaggcct     180

ggaaagggtc ttgagtggat gggacggatt tatcctggag atggagaaac tatctacaat     240

gggaaattca gggtcagagt cacgattacc gcggacgaat ccacgagcac agcctacatg     300

gagctgagca gcctgagatc tgaggacacg gccgtgtatt actgtgcgag aggctatgat     360

gattactcgt ttgcttactg gggccaggga accacggtca ccgtctcttc aggtggtggt     420

ggatccggag gtggtggatc gggtggtgga ggatcggata ttgtgatgac tcagtctcca 480

ctctccctgc ccgtcacccc tggagagccg gcctccatct cctgcaggtc tagtaagagt 540

ctcctgcata gtaatggcaa cacttacttg tattggttcc agcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat gggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc agcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 24
<211> 524
<212> PRT
<213> Artificial

<220>
<223> an artificially synthesized peptide sequence

<400> 24
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Met Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly

        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Gln Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro

305                 310                 315                 320

Gly Lys Gly Leu Glu Trp Met Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Gln Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro

500                 505                 510

Phe Thr Phe Gly Gln Gly Thr Lys Leu Glu Ile Lys
        515                 520

<210>  25
<211>  1572
<212>  DNA
<213>  Artificial

<220>
<223>  an artificially synthesized nucleotide sequence

<400>  25
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actgggtgag gcagaggcct     180

ggaaagggtc ttgagtgggt tggacggatt tatcctggag atggagaaac tatctacaat     240

gggaaattca gggtcagagt cacgattacc gcggacgaat ccacgagcac agcctacatg     300

aggctgagca gcctgagatc tgaggacacg gccgtgtatt actgtgcgag aggctatgat     360

gattactcgt ttgcttactg gggccaggga accacggtca ccgtctcttc aggtggtggt     420

ggatccggag gtggtggatc gggtggtgga ggatcggata ttgtgatgac tcaggctgca     480

ctctccctgc ccgtcacccc tggagagccg gcctccatct cctgcaggtc tagtaagagt     540

ctcctgcata gtaatggcaa cacttacttg tattggtacc tgcagaagcc agggcagtct     600

ccacagctcc tgatctatcg gatgtccaac cttgcctcag gggtccctga caggttcagt     660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtgggt tggacggatt tatcctggag atggagaaac tatctacaat 1020

gggaaattca gggtcagagt cacgattacc gcggacgaat ccacgagcac agcctacatg 1080

aggctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcaggctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 26
<211> 524

<212>  PRT
<213>  Artificial

<220>
<223>  an artificially synthesized peptide sequence

<400>  26
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Val Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Arg Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ala Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Val Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Arg Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ala Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  27

<211> 1572
<212> DNA
<213> Artificial

<220>
<223> an artificially synthesized nucleotide sequence

<400> 27
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtgggt tggacggatt tatcctggag atggagaaac tatctacaat 240

gggaaattca ggggcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcaggctgca 480

ctctccctgc ccgtcacccc tggagagccg gcctccatct cctgcaggtc tagtaagagt 540

ctcctgcata gtaatggcaa cacttacttg tattggtacc tgcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtgggt tggacggatt tatcctggag atggagaaac tatctacaat 1020

gggaaattca ggggcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcaggctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 28
<211> 524
<212> PRT
<213> Artificial

<220>
<223> an artificially synthesized peptide sequence

<400> 28

Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Val Gly Arg Ile Tyr Pro Gly Asp Gly Glu Thr Ile Tyr Asn
65                  70                  75                  80

Gly Lys Phe Arg Gly Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ala Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Val Gly Arg Ile Tyr Pro Gly Asp Gly Glu
                325                 330                 335

Thr Ile Tyr Asn Gly Lys Phe Arg Gly Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ala Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  29
<211>  1572
<212>  DNA
<213>  Artificial

<220>
<223>  an artificially synthesized nucleotide sequence

<400> 29

atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtggat gggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctcca 480

ctctccctgc ccgtcacccc tggagagccg gcctccatct cctgcaggtc tagtaagagt 540

ctcctgcata gtaatggcaa cacttacttg tattggttcc agcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat gggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc agcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 30
<211> 524
<212> PRT
<213> Artificial

<220>
<223> an artificially synthesized peptide sequence

<400> 30
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe

        35                  40                  45

Thr Asn Ser Trp Met Asn Trp Val Arg Gln Arg Pro Gly Lys Gly Leu
    50                  55                  60

Glu Trp Met Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val

225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Met Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser

420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  31
<211>  1602
<212>  DNA
<213>  Artificial

<220>
<223>  an artificially synthesized nucleotide sequence

<400>  31
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actgggtgag gcagaggcct     180

ggaaagggtc ttgagtgggt tggacggatt tatcctggag atggagaaac tatctacaat 240

gggaaattca gggtcagagt cacgattacc gcggacgaat ccacgagcac agcctacatg 300

aggctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctgca 480

ctctccctgc ccgtcacccc tggagagccg gcctccatct cctgcaggtc tagtaagagt 540

ctcctgcata gtaatggcaa cacttacttg tattggtacc tgcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtgggt tggacggatt tatcctggag atggagaaac tatctacaat 1020

gggaaattca gggtcagagt cacgattacc gcggacgaat ccacgagcac agcctacatg 1080

aggctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aagactacaa ggatgacgac gataagtgat aa 1602

<210> 32
<211> 524
<212> PRT
<213> Artificial

<220>
<223> an artificially synthesized peptide sequence

<400> 32
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Val Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Arg Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ala Ala
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Tyr Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Ala Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Val Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Arg Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ala Ala Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Tyr Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Ala Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  33
<211>  1572
<212>  DNA
<213>  Artificial

<220>
<223>  an artificially synthesized nucleotide sequence

<400>  33
atggactgga cctggaggtt cctctttgtg gtggcagcag ctacaggtgt ccagtcccag      60

gtgcagctgg tgcagtctgg acctgaggtg aagaagcctg gggcctcagt gaaggtctcc     120

tgcaaggctt ctggatacac cttcaccaac tcctggatga actgggtgag gcagaggcct     180

ggaaagggtc ttgagtggat tggacggatt tatcctggag atggagaaac tatctacaat     240

gggaaattca gggtcagagt cacgattacc gcggacgaat ccacgagcac agcctacatg     300

gagctgagca gcctgagatc tgaggacacg gccgtgtatt actgtgcgag aggctatgat     360

gattactcgt ttgcttactg gggccaggga accacggtca ccgtctcttc aggtggtggt     420

ggatccggag gtggtggatc gggtggtgga ggatcggata ttgtgatgac tcagtctcca 480

ctctccctgc ccgtcacccc tggagagccg gcctccatct cctgcaggtc tagtaagagt 540

ctcctgcata gtaatggcaa cacttacttg tattggttcc tgcagaagcc agggcagtct 600

ccacagctcc tgatctatcg gatgtccaac cttgcctcag gggtccctga caggttcagt 660

ggcagtggat caggcacaga ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc tgcagaagcc agggcagtct 1380

ccacagctcc tgatctatcg gatgtccaac cttgcctcag gggtccctga caggttcagt 1440

ggcagtggat caggcacaga ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 34
<211> 524
<212> PRT
<213> Artificial

<220>
<223> an artificially synthesized peptide sequence

<400> 34
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Leu Gln Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Asp Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Gln Arg Pro
305                 310                 315                 320

Gly Lys Gly Leu Glu Trp Ile Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Leu Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Asp Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210> 35
<211> 1572
<212> DNA
<213> Artificial

<220>
<223> an artificially synthesized nucleotide sequence

<400> 35
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtggat tggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctcca 480

ctctccctgc ccgtcacccc tggagagccg gcctccatct cctgcaggtc tagtaagagt 540

ctcctgcata gtaatggcaa cacttacttg tattggttcc agcagaagcc agggcagtct 600

ccacagctcc tgatctatcg gatgtccaac cttgcctcag gggtccctga caggttcagt 660

ggcagtggat caggcacaga ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc agcagaagcc agggcagtct 1380

ccacagctcc tgatctatcg gatgtccaac cttgcctcag gggtccctga caggttcagt 1440

ggcagtggat caggcacaga ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 36

<211>  524
<212>  PRT
<213>  Artificial

<220>
<223>  an artificially synthesized peptide sequence

<400>  36
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro

145                     150                     155                     160

Leu Ser Leu Pro Val Thr Pro Gly Glu Pro Ala Ser Ile Ser Cys Arg
                165                     170                     175

Ser Ser Lys Ser Leu Leu His Ser Asn Gly Asn Thr Tyr Leu Tyr Trp
            180                     185                     190

Phe Gln Gln Lys Pro Gly Gln Ser Pro Gln Leu Leu Ile Tyr Arg Met
        195                     200                     205

Ser Asn Leu Ala Ser Gly Val Pro Asp Arg Phe Ser Gly Ser Gly Ser
    210                     215                     220

Gly Thr Asp Phe Thr Leu Lys Ile Ser Arg Val Glu Ala Glu Asp Val
225                     230                     235                     240

Gly Val Tyr Tyr Cys Met Gln His Ile Glu Tyr Pro Phe Thr Phe Gly
                245                     250                     255

Gln Gly Thr Lys Leu Glu Ile Lys Gly Gly Gly Gly Ser Gly Gly Gly
            260                     265                     270

Gly Ser Gly Gly Gly Gly Ser Gln Val Gln Leu Val Gln Ser Gly Pro
        275                     280                     285

Glu Val Lys Lys Pro Gly Ala Ser Val Lys Val Ser Cys Lys Ala Ser
    290                     295                     300

Gly Tyr Thr Phe Thr Asn Ser Trp Met Asn Trp Val Arg Gln Arg Pro
305                     310                     315                     320

Gly Lys Gly Leu Glu Trp Ile Gly Arg Ile Tyr Pro Gly Asp Gly Glu
                325                     330                     335

Thr Ile Tyr Asn Gly Lys Phe Arg Val Arg Val Thr Ile Thr Ala Asp

            340                 345                 350

Glu Ser Thr Ser Thr Ala Tyr Met Glu Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Gln Gln Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Asp Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210> 37
<211> 15
<212> PRT
<213> Artificial

<220>
<223> An artificially synthesized linker sequence

<400> 37
Gly Gly Gly Gly Ser Gly Gly Gly Gly Ser Gly Gly Gly Gly Ser
1               5                   10                  15

<210> 38
<211> 1572
<212> DNA
<213> Mus musculus

<400> 38
atggactgga cctggaggtt cctctttgtg gtggcagcag ctacaggtgt ccagtcccag     60

gtgcagctgg tgcagtctgg acctgaggtg aagaagcctg gggcctcagt gaaggtctcc    120

tgcaaggctt ctggatacac cttcaccaac tcctggatga actgggtgag ggagaggcct    180

ggaaagggtc ttgagtggat tggacggatt tatcctggag atggagaaac tatctacaat    240

gggaaattca gggtcagagt cacgattacc gcggacgaat ccacgagcac agcctacatg    300

caactgagca gcctgagatc tgaggacacg gccgtgtatt actgtgcgag aggctatgat    360

gattactcgt ttgcttactg gggccaggga accacggtca ccgtctcttc aggtggtggt    420

ggatccggag gtggtggatc gggtggtgga ggatcggata ttgtgatgac tcagtctcca    480

ctctccctgc ccgtcacccc tggagagccg gcctccatct cctgcaggtc tagtaagagt    540

ctcctgcata gtaatggcaa cacttacttg tattggttcc tggagaagcc agggcagtct 600

ccacagctcc tgatctatcg gatgtccaac cttgcctcag gggtccctga caggttcagt 660

ggcagtggat caggcacaga ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gaagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

caactgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc tgaagaagcc agggcagtct 1380

ccacagctcc tgatctatcg gatgtccaac cttgcctcag gggtccctga caggttcagt 1440

ggcagtggat caggcacaga ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 39
<211> 524
<212> PRT
<213> Mus musculus

<400> 39
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Glu Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Gln Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro

145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Leu Glu Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Asp Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Lys Arg Pro
305                 310                 315                 320

Gly Lys Gly Leu Glu Trp Ile Gly Arg Ile Tyr Pro Gly Asp Gly Glu
                325                 330                 335

Thr Ile Tyr Asn Gly Lys Phe Arg Val Arg Val Thr Ile Thr Ala Asp

            340                 345                 350

Glu Ser Thr Ser Thr Ala Tyr Met Gln Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Leu Lys Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Asp Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210> 40
<211> 1572
<212> DNA
<213> Mus musculus

<400> 40
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag ggagaggcct 180

ggaaagggtc ttgagtggat tggacggatt tatcctggag atggagaaac tatctacaat 240

gggaaattca gggtcagagt cacgattacc gcggacgaat ccacgagcac agcctacatg 300

caactgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctcca 480

ctctccctgc ccgtcacccc tggagagccg gcctccatct cctgcaggtc tagtaagagt 540

ctcctgcata gtaatggcaa cacttacttg tattggttcc tgaagaagcc agggcagtct 600

ccacagctcc tgatctatcg gatgtccaac cttgcctcag gggtccctga caggttcagt 660

ggcagtggat caggcacaga ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gaagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

caactgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc tggagaagcc agggcagtct 1380

ccacagctcc tgatctatcg gatgtccaac cttgcctcag gggtccctga caggttcagt 1440

ggcagtggat caggcacaga ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 41
<211> 524
<212> PRT
<213> Mus musculus

<400> 41
Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Glu Arg Pro Gly Lys Gly Leu
    50                  55                  60

Glu Trp Ile Gly Arg Ile Tyr Pro Gly Asp Gly Glu Thr Ile Tyr Asn
65                  70                  75                  80

Gly Lys Phe Arg Val Arg Val Thr Ile Thr Ala Asp Glu Ser Thr Ser
                85                  90                  95

Thr Ala Tyr Met Gln Leu Ser Ser Leu Arg Ser Glu Asp Thr Ala Val
            100                 105                 110

Tyr Tyr Cys Ala Arg Gly Tyr Asp Asp Tyr Ser Phe Ala Tyr Trp Gly
        115                 120                 125

Gln Gly Thr Thr Val Thr Val Ser Ser Gly Gly Gly Gly Ser Gly Gly
    130                 135                 140

Gly Gly Ser Gly Gly Gly Gly Ser Asp Ile Val Met Thr Gln Ser Pro
145                 150                 155                 160

Leu Ser Leu Pro Val Thr Pro Gly Glu Pro Ala Ser Ile Ser Cys Arg
                165                 170                 175

Ser Ser Lys Ser Leu Leu His Ser Asn Gly Asn Thr Tyr Leu Tyr Trp
            180                 185                 190

Phe Leu Lys Lys Pro Gly Gln Ser Pro Gln Leu Leu Ile Tyr Arg Met
        195                 200                 205

Ser Asn Leu Ala Ser Gly Val Pro Asp Arg Phe Ser Gly Ser Gly Ser
    210                 215                 220

Gly Thr Asp Phe Thr Leu Lys Ile Ser Arg Val Glu Ala Glu Asp Val
225                 230                 235                 240

Gly Val Tyr Tyr Cys Met Gln His Ile Glu Tyr Pro Phe Thr Phe Gly
                245                 250                 255

Gln Gly Thr Lys Leu Glu Ile Lys Gly Gly Gly Gly Ser Gly Gly Gly
            260                 265                 270

Gly Ser Gly Gly Gly Gly Ser Gln Val Gln Leu Val Gln Ser Gly Pro
        275                 280                 285

Glu Val Lys Lys Pro Gly Ala Ser Val Lys Val Ser Cys Lys Ala Ser
    290                 295                 300

Gly Tyr Thr Phe Thr Asn Ser Trp Met Asn Trp Val Arg Lys Arg Pro
305                 310                 315                 320

Gly Lys Gly Leu Glu Trp Ile Gly Arg Ile Tyr Pro Gly Asp Gly Glu
                325                 330                 335

Thr Ile Tyr Asn Gly Lys Phe Arg Val Arg Val Thr Ile Thr Ala Asp
            340                 345                 350

Glu Ser Thr Ser Thr Ala Tyr Met Gln Leu Ser Ser Leu Arg Ser Glu
        355                 360                 365

Asp Thr Ala Val Tyr Tyr Cys Ala Arg Gly Tyr Asp Asp Tyr Ser Phe
    370                 375                 380

Ala Tyr Trp Gly Gln Gly Thr Thr Val Thr Val Ser Ser Gly Gly Gly
385                 390                 395                 400

Gly Ser Gly Gly Gly Gly Ser Gly Gly Gly Gly Ser Asp Ile Val Met
                405                 410                 415

Thr Gln Ser Pro Leu Ser Leu Pro Val Thr Pro Gly Glu Pro Ala Ser
            420                 425                 430

Ile Ser Cys Arg Ser Ser Lys Ser Leu Leu His Ser Asn Gly Asn Thr
        435                 440                 445

Tyr Leu Tyr Trp Phe Leu Glu Lys Pro Gly Gln Ser Pro Gln Leu Leu
    450                 455                 460

Ile Tyr Arg Met Ser Asn Leu Ala Ser Gly Val Pro Asp Arg Phe Ser
465                 470                 475                 480

Gly Ser Gly Ser Gly Thr Asp Phe Thr Leu Lys Ile Ser Arg Val Glu
                485                 490                 495

Ala Glu Asp Val Gly Val Tyr Tyr Cys Met Gln His Ile Glu Tyr Pro
            500                 505                 510

Phe Thr Phe Gly Gln Gly Thr Lys Leu Glu Ile Lys
        515                 520

<210>  42
<211>  4
<212>  PRT
<213>  Artificial

<220>
<223>  An artificially synthesized peptide sequence

<400>  42
Gly Gly Gly Ser
1

<210> 43
<211> 4
<212> PRT
<213> Artificial

<220>
<223> An artificially synthesized peptide sequence

<400> 43
Ser Gly Gly Gly
1

<210> 44
<211> 5
<212> PRT
<213> Artificial

<220>
<223> An artificially synthesized peptide sequence

<400> 44
Gly Gly Gly Gly Ser
1               5

<210> 45
<211> 5
<212> PRT
<213> Artificial

<220>
<223> An artificially synthesized peptide sequence

<400> 45
Ser Gly Gly Gly Gly

1                5

<210>  46
<211>  6
<212>  PRT
<213>  Artificial

<220>
<223>  An artificially synthesized peptide sequence

<400>  46
Gly Gly Gly Gly Gly Ser
1                5

<210>  47
<211>  6
<212>  PRT
<213>  Artificial

<220>
<223>  An artificially synthesized peptide sequence

<400>  47
Ser Gly Gly Gly Gly Gly
1                5

<210>  48
<211>  7
<212>  PRT
<213>  Artificial

<220>
<223>  An artificially synthesized peptide sequence

<400>  48
Gly Gly Gly Gly Gly Gly Ser
1               5

<210>  49
<211>  7
<212>  PRT
<213>  Artificial

<220>
<223>  An artificially synthesized peptide sequence

<400>  49
Ser Gly Gly Gly Gly Gly Gly
1               5